(19) Europäisches Patentamt
European Patent Office
Office européen des brevets

(11) **EP 4 306 656 B1**

(12) **EUROPEAN PATENT SPECIFICATION**

(45) Date of publication and mention of the grant of the patent:
**02.09.2026 Bulletin 2026/36**

(21) Application number: **22766717.7**

(22) Date of filing: **09.02.2022**

(51) International Patent Classification (IPC):
***C12Q 1/6886*** *(2018.01)* ***C12N 15/09*** *(2006.01)*
***C12Q 1/686*** *(2018.01)* ***C12Q 1/6809*** *(2018.01)*
***C12Q 1/6811*** *(2018.01)*

(52) Cooperative Patent Classification (CPC):
(C-Sets available)
**C12Q 1/6886; C12N 15/09;** C12Q 1/6811;
C12Q 2600/112; C12Q 2600/154 (Cont.)

(86) International application number:
**PCT/JP2022/005142**

(87) International publication number:
**WO 2022/190752 (15.09.2022 Gazette 2022/37)**

(54) **CANCER TEST REAGENT SET, METHOD FOR PRODUCING CANCER TEST REAGENT SET, AND CANCER TEST METHOD**

KREBSTESTREAGENZIENSET, VERFAHREN ZUR HERSTELLUNG EINES KREBSTESTREAGENZIENSETS UND KREBSTESTVERFAHREN

ENSEMBLE DE RÉACTIFS POUR LE DÉPISTAGE DU CANCER, PROCÉDÉ DE FABRICATION D'UN ENSEMBLE DE RÉACTIFS POUR LE DÉPISTAGE DU CANCER, ET PROCÉDÉ DE DÉPISTAGE DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2021 JP 2021040758**

(43) Date of publication of application:
**17.01.2024 Bulletin 2024/03**

(73) Proprietor: **FUJIFILM Corporation**
**Tokyo 106-8620 (JP)**

(72) Inventor: **WAKITA Maiko**
**Ashigarakami-gun, Kanagawa 258-8538 (JP)**

(74) Representative: **Dehns Germany Partnerschaft mbB**
**Theresienstraße 6-8**
**80333 München (DE)**

(56) References cited:
**WO-A1-2020/163403**
**WO-A2-2020/227127**
**CN-A- 109 680 060**
**JP-A- 2016 015 963**
**US-A1- 2020 255 904**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
C12Q 1/6811, C12Q 2523/125, C12Q 2537/149, C12Q 2537/159

## Description

BACKGROUND OF THE INVENTION

1. Field of the Invention

The present disclosure relates to a cancer examination reagent set, a production method for a cancer examination reagent set, and a cancer examination method.

2. Description of the Related Art

JP2020-096601A discloses a method of determining polyploidy of a chromosome segment contained in a specimen obtained from an individual conceived to have cancer, where the method includes determining whether or not copy number diversity exists in tumor cells of the individual.

JP2009-009396A discloses a medical examination information management system that determines whether or not to carry out the main medical examination based on the sensitivity and specificity of the preliminary medical examination.

WO2020227127A2 discloses methods for identifying and/or quantifying low abundance, nucleotide base mutations, insertions, deletions, translocations, splice variants, miRNA variants, alternative transcripts, alternative start sites, alternative coding sequences, alternative non-coding sequences, alternative splicings, exon insertions, exon deletions, intron insertions, or other rearrangement at the genome level and/or methylated or hydroxymethylated nucleotide bases, as well as markers to identify early cancer, monitor cancer treatment, and identify early cancer recurrence.

WO2020163403A1 discloses a hematological disorder (HD) assay panel for targeted detection of methylation patterns or variants specific to various hematological disorders.

US2020/255904A1 discloses cancer specific DNA methylation markers.

SUMMARY OF THE INVENTION

Early detection of cancer improves the prognosis. However, the early detection is not easy. The difficulty in the early detection depends on the kind of cancer. For example, regarding pancreatic cancer or hepatocellular cancer, the existing low invasive screening examinations are still less accurate, and regarding colon cancer or lung cancer, the burden of restraint time on a subject, financial burden, or physical burden is large, and thus the medical examination rate is low although there are recommended examinations. As a result, there is a demand for a cancer examination with high accuracy and a small burden on a subject.

DNA methylation analysis using DNA extracted from blood, urine, or stool as a specimen has attracted attention as a cancer examination that is low invasive. It is known that methylation or unmethylation of DNA occurs in association with carcinogenesis and that CpG sites where methylation or unmethylation occurs differ between cancer kinds. DNA methylation analysis is promising as an examination method for identifying even the primary lesion of cancer. However, there are a huge number of CpG sites in the human genome, and there are CpG sites common to a plurality of kinds of cancers in terms of methylation or unmethylation, which may make it difficult to discriminate the kind of cancer.

The present disclosure has been made under the above circumstances.

An object of the present disclosure is to provide a cancer examination reagent set which increases a reliability degree of a cancer examination and a production method for a cancer examination reagent set, as well as a cancer examination method having a high reliability degree.

Specific means for solving the above problems include the following aspects.

A first aspect of the present invention provides a cancer examination reagent set as claimed in claim 1.

A second aspect of the present invention provides a production method for a cancer examination reagent set including a primary examination reagent and a secondary examination reagent, as claimed in claim 2.

In the production method for a cancer examination reagent set according to the second aspect of the present invention, optionally the (c) is the following (c-1),

(c-1) excluding a part or all of the CpG sites selected for the CpG site set 1, from the CpG sites constituting the CpG site set P and selecting a part or all of remaining CpG sites to create each of ${}_{n}C_{2}$ kinds of CpG site sets 2 for discriminating two kinds among the n kinds of cancers.

According to a third aspect of the present invention, a cancer examination method is provided as claimed in claim 5.

According to the present disclosure, there are provided a cancer examination reagent set which increases a reliability degree of a cancer examination and a production method for a cancer examination reagent set, as well as a

cancer examination method having a high reliability degree.

DESCRIPTION OF THE PREFERRED EMBODIMENTS

Hereinafter, embodiments according to the present disclosure will be described. These descriptions and Examples below are only illustrative of the embodiments and do not limit the ranges of the embodiments.

The term "step" in the present disclosure not only includes an independent step but also includes a step that may not be clearly distinguished from the other step but still achieves a desired effect of the step.

In the present disclosure, a numerical range expressed using "to" indicates a range including numerical values before and after "to" as a minimum value and a maximum value.

In the numerical ranges described stepwise in the present disclosure, the upper limit value or the lower limit value described in one numerical range may be replaced with the upper limit value or the lower limit value of the numerical range described stepwise in other stages. Further, in the numerical ranges described in the present disclosure, the upper limit value or the lower limit value of the numerical range may be replaced with the value shown in Examples.

In the present disclosure, the sequencer is a term including a first generation sequencer (a capillary sequencer), a second generation sequencer (a next generation sequencer), a third generation sequencer, a fourth generation sequencer, and a sequencer to be developed in the future. The sequencer may be a capillary sequencer, may be a next generation sequencer, or may be another sequencer. The sequencer is preferably a next generation sequencer from the viewpoints of the speed of analysis, the large number of specimens that can be processed at one time, and the like. The next generation sequencer (NGS) refers to a sequencer that is classified by being contrasted with a capillary sequencer (called a first generation sequencer) using the Sanger method. At present, the most popular next generation sequencer is a sequencer of which the principle is to capture fluorescence or luminescence linked to a complementary strand synthesis by DNA polymerase or a complementary strand binding by DNA ligase and determine the base sequence. Specific examples thereof include MiSeq (MiSeq is a registered trade name, Illumina, Inc.), HiSeq 2000 (Illumina, Inc., HiSeq is a registered trade name), and Roche 454 (Roche, Ltd.).

<Cancer examination reagent set>

The cancer examination reagent set according to the present disclosure is a reagent set for analyzing a degree of methylation of DNA and contains a primary examination reagent and a secondary examination reagent.

The primary examination reagent is a primer specifically designed for analyzing a degree of methylation of each of all CpG sites constituting a CpG site set 1 selected for examining n kinds of cancers. n is a natural number of 3 or more.

The secondary examination reagent is a set of ${}_nC_2$ kinds of primers specifically designed for analyzing a degree of methylation of each of all CpG sites constituting ${}_nC_2$ kinds of CpG site sets 2, where the ${}_nC_2$ kinds are selected for respectively discriminating two kinds among the n kinds of cancers.

According to the cancer examination reagent set according to the present disclosure, in a case where the kind of cancer cannot be identified by the primary examination or in a case where the reliability degree of the primary examination is low, it is possible to identify the kind of cancer by carrying out the secondary examination based on the results of the primary examination. As a result, according to the cancer examination reagent set according to the present disclosure, it is possible to increase the reliability degree of the cancer examination.

Examples of the method of DNA methylation analysis, which is adopted in the cancer examination reagent set according to the present disclosure, include any known method. Examples of embodiments of the DNA methylation analysis include bisulfite sequencing, microarray, and methylation-specific PCR.

The outline of the bisulfite sequencing is as follows.

In a case where DNA is treated with a bisulfite reagent, unmethylated cytosine is converted to uracil, whereas methylated cytosine remains as cytosine. That is, by the bisulfite treatment, the modification state (the unmethylated or methylated state) of cytosine is converted into the information of sequence (uracil or cytosine) at a position thereof. Next, DNA is amplified according to polymerase chain reaction (PCR). In this process, uracil is converted to thymine. Next, the sequence of the amplification product is analyzed using a sequencer. By determining whether the base at the position to be analyzed is thymine or cytosine, it is possible to know the modification state (the unmethylated or methylated state) of cytosine at the position of interest in DNA.

However, the bisulfite sequencing has a drawback that primer design is difficult. This is because there can be two possible base sequences of the CpG site after the bisulfite treatment, UG and CG. In addition, in a case where conversion to uracil has been carried out in a large number of CpG sites, the DNA after the bisulfite treatment has long regions composed three bases other than cytosine, and thus it is difficult to increase the specificity of the primer.

As a result, in the bisulfite sequencing, the number of regions that can be measured simultaneously is limited due to the difficulty in primer design, and thus there may be a case where sufficient reliability degree cannot be ensured in an examination in which one kind of cancer is identified from a plurality of kinds of candidates.

On the other hand, according to the cancer examination reagent set according to the present disclosure, even in a case of adopting the bisulfite sequencing as a DNA methylation analysis method for the reagent set, it is possible to increase the reliability degree of cancer examination since a secondary examination is carried out based on the examination result of the primary examination.

Hereinafter, the elements of the cancer examination reagent set will be described in detail.

[n kinds of cancers]

n is a natural number of 3 or more. Examples of embodiments of n include natural numbers of 3 or more and 16 or less, and more specific examples thereof include 3, 4, 5, 6, 7, 8, 9, 10, 11, 12, 13, 14, 15, and 16.

The cancer included in the n kinds of cancers may be any cancer of which onset or progression correlates with methylation or unmethylation of a gene. In the present disclosure, the concept of a gene includes a transcription regulatory region of the gene.

The cancers included in the n kinds of cancers are, for example, colon cancer, pancreatic cancer, hepatocellular cancer, bile duct cancer, gallbladder cancer ovarian cancer, breast cancer, lung cancer, esophageal cancer, stomach cancer, renal cell carcinoma, ureteral cancer, bladder cancer, prostate cancer, cervical cancer, and uterine cancer. It is preferable to select the n kinds of cancers from the group of these cancers. The combination of cancers included in the n kinds of cancers is not limited.

[Degree of methylation of CpG site]

The CpG site is a two-base sequence in which guanine appears next to cytosine.

In the present disclosure, the CpG site is specified according to the coordinates of the Genome Reference Consortium Human Build 37 (GRCh37), which is an international reference sequence of the human genome.

The degree of methylation of a CpG site is a value calculated from a set of DNA fragments and is calculated for each CpG site. A degree of methylation of a certain CpG site is {the number of DNA fragments in which the CpG site is methylated/(the number of DNA fragments in which the CpG site is methylated + the number of DNA fragments in which the CpG site is unmethylated)}, and it is indicated in terms of percentage (%).

In the present disclosure, methylation of cytosine refers to the addition of a methyl group to the carbon at the 5-position of the pyrimidine ring of cytosine.

[CpG site set 1]

The CpG site set 1 is a CpG site set selected for the intended purpose of examining n kinds of cancers, and it includes a plurality of CpG sites.

The number of CpG sites constituting the CpG site set 1 is not limited. As the number of CpG sites constituting the CpG site set 1 increases, the accuracy of identifying the kind of cancer tends to increase. The lower limit of the number of CpG sites constituting the CpG site set 1 is, for example, 30 or more, 40 or more, 50 or more, or 60 or more.

The upper limit of the number of CpG sites constituting the CpG site set 1 may be set from the viewpoint of the time or cost required for the primary examination. The upper limit of the number of CpG sites constituting the CpG site set 1 is, for example, 600 or less, 500 or less, 400 or less, 300 or less, 200 or less, or 100 or less.

[Primary examination reagent]

The primary examination reagent is a reagent designed so that the degree of methylation of the CpG site set 1 can be comprehensively analyzed.

More specifically, the primary examination reagent includes a primer that is specifically designed for each of all CpG sites constituting the CpG site set 1 so that the CpG sites constituting the CpG site set 1 can be amplified.

It suffices that the primary examination reagent includes at least a primer specific to the CpG site set 1, among the reagents necessary for the DNA methylation analysis. The primary examination reagent may include all the reagents required for the DNA methylation analysis. The primary examination reagent may include an instrument used for the DNA methylation analysis.

The primary examination reagent is, for example, a reagent for bisulfite sequencing and it includes a primer group specific for the CpG site set 1. The primary examination reagent may include all reagents necessary for all steps of bisulfite treatment, multiplex PCR, and sequencing, and an example of an embodiment thereof includes a bisulfite reagent, a primer, a probe, a PCR reagent, and a sequencing reagent. Each of the bisulfite reagent, the primer, the probe, the PCR reagent, and the sequencing reagent may be a commercially available product or may be a newly prepared reagent.

[CpG site set 2]

The CpG site set 2 is a CpG site set selected for each pair of ${}_nC_2$ kinds, which are two kinds extracted for n kinds of cancers. As a result, there are ${}_nC_2$ kinds of the CpG site sets 2 in the secondary examination reagent. The ${}_nC_2$ kinds of the CpG site sets 2 differ from each other in at least a part of CpG sites. The ${}_nC_2$ kinds of the CpG site sets 2 may have the same number of CpG sites or may have the numbers of CpG sites different from each other.

The form described below is a form common to the ${}_nC_2$ kinds of the CpG site sets 2.

The CpG site set 2 is a CpG site set selected for the intended purpose of discriminating two kinds among the n kinds of cancers, and it includes a plurality of CpG sites.

The number of CpG sites constituting the CpG site set 2 is not limited. As the number of CpG sites constituting the CpG site set 2 increases, the accuracy of identifying the kind of cancer tends to increase. The lower limit of the number of CpG sites constituting the CpG site set 2 is, for example, 30 or more, 40 or more, 50 or more, or 60 or more.

The upper limit of the number of CpG sites constituting the CpG site set 2 may be set from the viewpoint of the time or cost required for the secondary examination. The upper limit of the number of CpG sites constituting the CpG site set 2 is, for example, 600 or less, 500 or less, 400 or less, 300 or less, 200 or less, or 100 or less.

The number of CpG sites constituting the CpG site set 2 may be smaller or may be smaller than the number of CpG sites constituting the CpG site set 1.

It is preferable that at least a part of CpG sites constituting the CpG site set 2 is different from CpG sites constituting the CpG site set 1.

It is more preferable that all CpG sites constituting the CpG site set 2 is different from CpG sites constituting the CpG site set 1.

In a case of the above-described form, the number of kinds of CpG sites that contribute to the identification of the kind of cancer through the primary examination and the secondary examination is increased, and thus the accuracy of identifying the kind of cancer can be improved.

From the above viewpoint, a preferred form of the secondary examination reagent will be described.

A set of ${}_nC_2$ kinds of reagents for analyzing a degree of methylation of each of ${}_nC_2$ kinds of CpG site sets 2, the ${}_nC_2$ kinds being selected for respectively discriminating two kinds among the n kinds of cancers. However, in each of the ${}_nC_2$ kinds of CpG site sets 2, a part or all of CpG sites constituting the CpG site set 2 are CpG sites that are not included in the CpG site set 1.

[Secondary examination reagent]

The secondary examination reagent is a reagent designed so that the degree of methylation of the CpG site set 2 can be comprehensively analyzed. The set of the ${}_nC_2$ kinds of reagents, which are designed for the respective ${}_nC_2$ kinds of the CpG site sets 2, is the secondary examination reagent. However, regardless of the kind of the CpG site set, elements common to the DNA methylation analysis may be shared between the ${}_nC_2$ kinds of reagents. In addition, regardless of the kind of the CpG site set, elements common to the DNA methylation analysis may be shared between the primary examination reagent and the secondary examination reagent.

Each of the ${}_nC_2$ kinds of reagents constituting the secondary examination reagent includes a primer that is specifically designed for each of all CpG sites constituting the CpG site set 2 so that the CpG sites constituting the CpG site set 2 can be amplified.

It suffices that the secondary examination reagent includes at least a primer specific to the CpG site set 2, among the reagents necessary for the DNA methylation analysis. The secondary examination reagent may include all the reagents required for the DNA methylation analysis. The secondary examination reagent may include an instrument used for the DNA methylation analysis.

The secondary examination reagent is, for example, a reagent for bisulfite sequencing and it includes a primer group specific for the CpG site set 2. The secondary examination reagent may include all reagents necessary for all steps of bisulfite treatment, multiplex PCR, and sequencing, and an example of an embodiment thereof includes a bisulfite reagent, a primer, a probe, a PCR reagent, and a sequencing reagent. Each of the bisulfite reagent, the primer, the probe, the PCR reagent, and the sequencing reagent may be a commercially available product or may be a newly prepared reagent.

<production method for cancer examination reagent set>

The production method for a cancer examination reagent set according to the present disclosure is suitable as a production method for the above-described cancer examination reagent set.

In the production method for a cancer examination reagent set according to the present disclosure, a reagent set including a primary examination reagent and a secondary examination reagent is produced.

The production method for a cancer examination reagent set according to the present disclosure includes the following (a) to (e) for the intended purpose of providing, in the primary examination reagent, an element (for example, a primer) for specifically and comprehensively analyzing the degree of methylation of the CpG site set 1, and providing, in the secondary examination reagent, an element (for example, a primer) for specifically and comprehensively analyzing the degree of methylation of the CpG site set 2.

(a) Creating a CpG site set P consisting of CpG sites having a significant difference in the degree of methylation in at least one kind among n kinds of cancers. n is a natural number of 3 or more.
(b) Selecting a part of the CpG sites constituting the CpG site set P to create a CpG site set 1.
(c) Selecting a part of the CpG sites constituting the CpG site set P to create each of ${}_nC_2$ kinds of CpG site sets 2 for discriminating two kinds among the n kinds of cancers.
(d) Producing a reagent for analyzing a degree of methylation of the CpG site set 1 to form the primary examination reagent.
(e) Producing ${}_nC_2$ kinds of reagents for analyzing degrees of methylation of the ${}_nC_2$ kinds of CpG site sets 2, respectively, to form the secondary examination reagent.

Hereinafter, (a), (b), (c), (d), and (e) are referred to as a step (a), a step (b), a step (c), a step (d), and a step (e), respectively. Hereinafter, elements of the production method for a cancer examination reagent set will be described in detail.

[Step (a)]

In the step (a), a CpG site set P serving as a parent set of the CpG site set 1 and the CpG site set 2 is created.
The CpG site set P is a CpG site set consisting of CpG sites having a significant difference in the degree of methylation in at least one kind among n kinds of cancers, and it includes a plurality of CpG sites.
The creation of the CpG site set P is carried out, for example, by comparing the data on the degree of methylation between n kinds of tumor tissues and normal tissue and then selecting CpG sites having a significant difference in the degree of methylation in at least one kind of tumor tissue.
Alternatively, the creation of the CpG site set P is carried out, for example, by comparing the data on the degree of methylation between n kinds of tumor tissues and normal tissue and then selecting CpG sites that have a high degree of methylation in at least one kind of tumor tissue and have a low degree of methylation in normal tissue. In other words, the CpG site set P, which consists of CpG sites having a high degree of methylation in at least one kind among n kinds of cancers, is created.
The data on the degree of methylation of the tumor tissue and the normal tissue may be obtained by actually collecting a tumor tissue from a cancer patient to carry out DNA analysis or may be obtained from the database open to the public. Examples of the public database that is open to the public include the Cancer Genome Atlas (TCGA).
The number of CpG sites constituting the CpG site set P is not limited. As the number of CpG sites constituting the CpG site set P increases, the number of CpG sites constituting each of the CpG site set 1 and the CpG site set 2 increases, and thus the accuracy of identifying the kind of cancer tends to increase. The lower limit of the number of CpG sites constituting the CpG site set P is, for example, 100 or more, 200 or more, 300 or more, 400 or more, or 500 or more.
The upper limit of the number of CpG sites constituting the CpG site set P may be set from the viewpoint of the time or cost required for the selection of each of the CpG site set 1 and the CpG site set 2. The upper limit of the number of CpG sites constituting the CpG site set P is, for example, 1,000 or less, 900 or less, 800 or less, 700 or less, or 600 or less.

[Step (b)]

In the step (b), CpG sites included in the CpG site set P are narrowed down to select the CpG site set 1.
The narrowing down of CpG sites for selecting the CpG site set 1 is carried out, for example, by checking the following items.
That is, whether it is possible to design a primer, whether DNA is amplified by single-plex PCR using the designed primer, and whether DNA is amplified by multiplex PCR using the designed primer.
For the primer design, a known design program such as Primer Suite may be used, or an original design program may be used.

[Step (c)]

In the step (c), CpG sites included in the CpG site set P are narrowed down to select the CpG site set 2. The selection of the CpG site set 2 is carried out for each pair of ${}_nC_2$ kinds, which are two kinds extracted for n kinds of cancers.

The ${}_{n}C_{2}$ kinds of the CpG site sets 2 differ from each other in at least a part of CpG sites. The ${}_{n}C_{2}$ kinds of the CpG site sets 2 may have the same number of CpG sites or may have the numbers of CpG sites different from each other.

The form described below is a form common to the ${}_{n}C_{2}$ kinds of the CpG site sets 2.

The narrowing down of CpG sites for selecting the CpG site set 2 is carried out, for example, by checking the following items.

That is, whether a significant difference in the degree of methylation between two kinds of cancers, whether it is possible to design a primer, whether DNA is amplified by single-plex PCR using the designed primer, and whether DNA is amplified by multiplex PCR using the designed primer.

For the primer design, a known design program such as Primer Suite may be used, or an original design program may be used.

Regarding the selection of the CpG site set 2, from the viewpoint that the number of kinds of CpG sites contributing to the identification of the kind of cancer through the primary examination and the secondary examination is increased, it is preferable to exclude a part or all of the CpG sites selected for the CpG site set 1, from the CpG sites constituting the CpG site set P, and then select CpG sites constituting the CpG site set 2 from the remaining CpG sites. As a result, CpG sites that are not the analysis targets in the primary examination become analysis targets in the secondary examination, and thus it is possible to increase the accuracy of cancer examination.

From the above viewpoint, a step (c-1) and a step (c-2), which are preferred forms of the step (c), will be described.

Step (c-1): Excluding a part or all of the CpG sites selected for the CpG site set 1, from the CpG sites constituting the CpG site set P and selecting a part or all of remaining CpG sites to create each of the ${}_{n}C_{2}$ kinds of CpG site sets 2 for discriminating two kinds among the n kinds of cancers.

Step (c-2): Selecting a part or all of CpG sites that have not been selected for the CpG site set 1, from the CpG sites constituting the CpG site set P, and creating each of the ${}_{n}C_{2}$ kinds of CpG site sets 2 for discriminating two kinds among the n kinds of cancers.

[Step (d)]

In the step (d), as the primary examination reagent, a reagent for specifically and comprehensively analyzing the degree of methylation of the CpG site set 1 is produced.

Hereinafter, an embodiment of the step (d) will be described.

A primer specific for each of all CpG sites constituting the CpG site set 1 is produced. The manufactured primer is accommodated in one container, and this is used as the primary examination reagent.

The specific primer for each CpG site may be a primer designed in the step (b) or may be a newly designed primer.

The step (d) may be a step of producing at least an element (for example, a primer) specific to the CpG site set 1, among the reagents necessary for the DNA methylation analysis. The step (d) may be a step of producing all the reagents necessary for the DNA methylation analysis.

[Step (e)]

In the step (e), as the secondary examination reagent, a reagent for specifically and comprehensively analyzing the degree of methylation of the CpG site set 2 is produced. The ${}_{n}C_{2}$ kinds of reagents for the respective ${}_{n}C_{2}$ kinds of CpG site sets 2 are produced, and the produced ${}_{n}C_{2}$ kinds of reagents are made into a set to form the secondary examination reagent.

Hereinafter, an embodiment of the step (e) will be described.

A primer specific for each of all CpG sites constituting the CpG site set 2 is produced. At this time, a primer is produced for each of the ${}_{n}C_{2}$ kinds of CpG site sets 2. Each of the produced primers is accommodated in one container for each of the ${}_{n}C_{2}$ kinds of CpG site sets 2, and containers containing ${}_{n}C_{2}$ kinds of primers are collected to be used as the secondary examination reagent.

The specific primer for each CpG site may be a primer designed in the step (c) or may be a newly designed primer.

The step (e) may be a step of producing at least an element (for example, a primer) specific to the CpG site set 2, among the reagents necessary for the DNA methylation analysis. The step (e) may be a step of producing all the reagents necessary for the DNA methylation analysis.

The production method for a cancer examination reagent set according to the present disclosure includes assembling, as a set, the primary examination reagent and the secondary examination reagent.

Between the step (a), the step (b), and the step (c), as well as between the step (d) and the step (e), there may be a case where temporal or spatial difference is large as compared with between other steps. Examples of such a case include an embodiment in which the step (a), the step (b), and the step (c) are carried out in an experimental facility or a research facility, and the step (d) and the step (e) are carried out in a manufacturing facility.

<Cancer examination method>

The subject of the cancer examination method according to the present disclosure is a human. The subject is, for example, an examinee who has undergone a medical examination according to his/her own will, or a person who has been suspected of having cancer at a medical institution.

The information obtained by the cancer examination method according to the present disclosure is useful as information that assists the diagnosis of a doctor, a basis for a doctor or a subject to determine the necessity of a detailed examination (for example, an imaging test), a basis for a doctor to select a medical treatment method or a therapeutic drug, a motivation for a subject to improve his/her lifestyle habit, or the like.

The DNA, which is a specimen of the cancer examination method according to the present disclosure, is obtained from a biological specimen of a subject. The biological specimen is, for example, preferably blood, urine, stool, saliva, cerebrospinal fluid, pericardial fluid, pleural effusion, or ascites. It is known that circulating tumor DNA (ctDNA) released from cancer cells or tumor cells is present in these biological specimens, which is a very versatile biological specimen for a plurality of kinds of cancers. The biological specimen is preferably blood, urine, excrement, or saliva from the viewpoint of low invasiveness to a subject, and it is preferably blood from the viewpoint that the concentration of ctDNA is relatively high and from the viewpoint that ctDNA of various kinds of cancers can be contained.

In the present disclosure, the blood includes blood itself and blood diluted with physiological saline; stored blood obtained by adding additives such as glucose and an anticoagulant agent to blood; fractions thereof (for example, blood plasma and serum); and the like.

The extraction of the DNA from the biological specimen may be carried out by extracting DNA from cells contained in the biological specimen or may be carried out by extracting cell-free DNA (cfDNA) contained in the biological specimen.

The cancer examination method according to the present disclosure is a cancer examination method using the cancer examination reagent set according to the present disclosure, and it includes the following (1) to (3).

(1) A primary examination for examining n kinds of cancers using the primary examination reagent. n is a natural number of 3 or more.

(2) Determining necessity of a secondary examination based on results of the primary examination, and selecting, in descending order of degree of certainty, two kinds of cancers detected in the primary examination, in a case where the secondary examination is required.

(3) A secondary examination for discriminating the two kinds of cancers using the secondary examination reagent.

The cancer examination method according to the present disclosure is an examination method that makes it possible to examine a plurality of kinds of cancers, and it is a simple examination method for analyzing the degree of methylation of DNA in both the primary examination and the secondary examination. According to the cancer examination method according to the present disclosure, it is possible to obtain an examination result with high reliability degree since a secondary examination is carried out based on the examination result of the primary examination.

Hereinafter, (1), (2), and (3) will be referred to as a step (1), a step (2), and a step (3), respectively. Hereinafter, elements of the cancer examination method will be described in detail.

[Step (1)]

The step (1) is carried out using n kinds of cancers as examination targets and using the primary examination reagent.

Hereinafter, an embodiment of the step (1) will be described. However, the cancer examination method according to the present disclosure is not limited to this.

The blood of a subject is collected, blood plasma is separated, and cfDNA is extracted from the blood plasma. A general DNA extraction reagent such as QIA amplifier Circulating Nucleic Acid (QIAGEN N.V.) may be used for the extraction of cfDNA. It is preferable to confirm by electrophoresis that the extracted cfDNA is not contaminated with genomic DNA from leukocytes or the like.

Using the extracted DNA as a specimen, the degree of methylation of the CpG site set 1 is comprehensively analyzed using the primary examination reagent.

The method of DNA methylation analysis is, for example, bisulfite sequencing. The PCR for the bisulfite sequencing is carried out by multiplex PCR. It is preferable to use NGS for the sequencing. The sequence information obtained by the sequencing is analyzed using software capable of processing a bisulfite conversion sequence such as Bismark.

The estimation of the presence or absence of cancer and the kind of cancer from the DNA methylation information of the subject is realized, for example, by preparing in advance a classifier covering the degree of methylation of the CpG

site set 1, and collating the classifier with the methylation information of the subject. From the viewpoint of increasing the reliability degree of the estimation, a preferred form is to collate the methylation information of the subject with a plurality of kinds of classifiers.

The estimation of the kind of cancer with a classifier can be executed by machine learning. Examples of the machine learning include a support vector machine, a regression analysis using a random forest, a logistic regression analysis, neural networking, naive bays, and a decision tree.

For example, the classifier is constructed from the data of DNA methylation analysis obtained from tumor tissues of a plurality of cancer patients or constructed from the cancer DNA methylation information open to the public.

[Step (2)]

The step (2) includes determining necessity of a secondary examination based on results of the primary examination, and selecting, in descending order of degree of certainty, two kinds of cancers detected in the primary examination, in a case where the secondary examination is required.

Hereinafter, an embodiment of the step (2) will be described. However, the cancer examination method according to the present disclosure is not limited to this.

A threshold value of the degree of certainty is set in advance for the estimation of the kind of cancer with the classifier for the primary examination.

**In** a case where the degree of certainty of cancer A is equal to or higher than the threshold value and the degree of certainty of other cancers is lower than the threshold value, the secondary examination is determined to be unnecessary, and the subject is determined to suffer from the cancer A.

**In** a case where the degree of certainty of the cancer A is the highest and is close to the threshold value although being equal to or higher than the threshold value, and the degree of certainty of cancer B is second highest and is close to the threshold value although being lower than the threshold value, the secondary examination is determined to be necessary, and the cancer A and the cancer B are selected as examination targets for the secondary examination.

**In** a case where the degree of certainty of all kinds of cancers is lower than the threshold value, the secondary examination is determined to be necessary, and two kinds of cancers are selected as examination targets for the secondary examination in descending order of the degree of certainty estimated by the classifier.

**In** a case where the degree of certainty of all kinds of cancers is lower than the threshold value and is a value far from the threshold value, the secondary examination is determined to be unnecessary, and the subject is determined not to suffer from any one of the n kinds of cancers.

[Step (3)]

The step (3) is a secondary examination for discriminating the two kinds of cancers using the secondary examination reagent, and it is carried out using the secondary examination reagent.

The secondary examination is an examination that re-evaluates the two kinds estimated to be most likely to be affected in the primary examination.

The CpG site set 2 to be analyzed in the secondary examination is one kind of the ${}_nC_2$ kinds, and it is the CpG site set 2 for discriminating the two kinds of cancers selected in the step (2). Therefore, one kind (which is the reagent for the CpG site set 2 to be analyzed) is selected from the ${}_nC_2$ kinds of reagents included in the secondary examination reagent and is used for the secondary examination.

Hereinafter, an embodiment of the step (3) will be described. However, the cancer examination method according to the present disclosure is not limited to this.

Using DNA of a subject as a specimen, the degree of methylation of the selected one kind of the CpG site set 2 is comprehensively analyzed using the secondary examination reagent. The DNA as the specimen may be the DNA remaining in the step (1) or may be the DNA newly extracted from the biological specimen of the subject.

The method of DNA methylation analysis is, for example, bisulfite sequencing. The PCR for the bisulfite sequencing is carried out by multiplex PCR. It is preferable to use NGS for the sequencing. The sequence information obtained by the sequencing is analyzed using software capable of processing a bisulfite conversion sequence such as Bismark.

The estimation of the kind of cancer from the DNA methylation information of the subject is realized, for example, by preparing in advance a classifier covering the degree of methylation of the CpG site set 2, for each of the ${}_nC_2$ kinds of the CpG site sets 2, and collating the classifier with the methylation information of the subject. From the viewpoint of increasing the reliability degree of the estimation, a preferred form is to collate the methylation information of the subject with a plurality of kinds of classifiers.

The method of estimating the kind of cancer with the classifier and the method of constructing the classifier are the same as those in the step (1).

Hereinafter, a determination example based on the results of the secondary examination will be described. However, the cancer examination method according to the present disclosure is not limited to this.

A threshold value of the degree of certainty is set in advance for the estimation of the kind of cancer with the classifier for the secondary examination.

In a case where the degree of certainty of cancer A is equal to or higher than the threshold value and the degree of certainty of cancer B is lower than the threshold value, the subject is determined to suffer from the cancer A.

In a case where the degree of certainty of the cancer A is close to the threshold value although being equal to or higher than the threshold value, and the degree of certainty of cancer B is close to the threshold value although being lower than the threshold value, it is determined that the subject may suffer from the cancer A and cancer B.

In a case where the degree of certainty of both the cancer A and the cancer B is lower than the threshold value and is a value far from the threshold value, the subject is determined not to suffer from any one of the n kinds of cancers.

Examples

Hereinafter, embodiments of the invention will be further described with reference to Examples. However, the embodiments of the invention are not limited to these Examples.

<Example 1: Manufacturing of cancer examination reagent set>

[Step (a)]

Colon cancer, pancreatic cancer, hepatocellular cancer, bile duct cancer, and ovarian cancer were selected as examination targets.

Data from 937 specimens were extracted from the public database of the Cancer Genome Atlas (TCGA), the data on the degree of methylation was compared between tumor tissues of five kinds of cancers and normal tissue (including healthy cfDNA), and 500 CpG sites, having a high degree of methylation in at least one kind of tumor tissue and having a low degree of methylation in the normal tissue, were selected. The selected 500 CpG site set is referred to as a CpG site set P-5.

[Step (b)]

A primer specific for each of all CpG sites constituting the CpG site set P-5 was designed. For the primer design, Primer Suite, which is software for executing the primer design for the bisulfite multiplex PCR, was used. Regarding the primer that could be designed, it was checked whether or not DNA amplification could be carried out by single-plex PCR. Regarding the primer that enabled the DNA amplification by single-plex PCR, it was checked whether or not DNA amplification could be carried out by multiplex PCR in one tube. 61 CpG sites for which DNA amplification was confirmed by multiplex PCR were selected. The set of 61 CpG sites selected is referred to as a CpG site set 1-5. Table 1 shows the CpG site set 1-5.

The positions shown in Table 1 are positions on the coordinates of GRCh37. In a case where the CpG site is known to be included in a transcription region or a transcription regulatory region of a gene, the name of the gene is described in the table.

[Table 1]

| CpG site set 1-5 | | | | | |
|---|---|---|---|---|---|
| Chromosome | Position | Gene | Chromosome | Position | Gene |
| 21 | 36399258 | RUNX1 | 1 | 14026590 | PRDM2 |
| 10 | 97802941 | CCNJ | 2 | 37899953 | CDC42EP3 |
| 7 | 73508618 | LIMK1 | 7 | 27196302 | HOXA7 |
| 8 | 55370423 | SOX17 | 2 | 144695078 | |
| 19 | 57831909 | ZNF543 | 10 | 122708532 | |
| 16 | 67034882 | CES8 | 10 | 113943887 | GPAM |
| 16 | 58498190 | NDRG4 | 7 | 96634660 | DLX6AS;DLX6 |
| 5 | 169724656 | LCP2 | 9 | 124461171 | DAB2IP |

(continued)

| CpG site set 1-5 | | | | | |
|---|---|---|---|---|---|
| Chromosome | Position | Gene | Chromosome | Position | Gene |
| 12 | 117798954 | NOS1 | 14 | 23356251 | REM2 |
| 12 | 105478345 | ALDH1L2 | 7 | 27196365 | HOXA7 |
| 1 | 78511856 | GIPC2 | 5 | 43007632 | |
| 3 | 142682652 | PAQR9 | 9 | 130212635 | RPL12;LRSAM1 |
| 11 | 67351271 | GSTP1 | 7 | 16794078 | TSPAN13 |
| 1 | 29586414 | PTPRU | 14 | 37126902 | PAX9 |
| 1 | 143913847 | FAM72D | 6 | 152623304 | SYNE1 |
| 17 | 61524073 | CYB561 | 1 | 63795711 | |
| 15 | 90208810 | PLIN1 | 5 | 40681137 | PTGER4 |
| 1 | 151810893 | C2CD4D;LOC100132111 | 10 | 102792249 | PDZD7;SFXN3 |
| 1 | 29586299 | PTPRU | 5 | 178004204 | COL23A1 |
| 6 | 10887047 | SYCP2L | 3 | 168864101 | MECOM |
| 12 | 104850745 | CHST11 | 7 | 42267719 | GLI3 |
| 13 | 98628060 | IP05 | 16 | 215960 | HBM |
| 15 | 76633086 | ISL2 | 7 | 142986693 | CASP2 |
| 7 | 1272545 | UNCX | 2 | 162280519 | TBR1 |
| 17 | 75369939 | SEPT9 | 11 | 66624841 | PC;LRFN4 |
| 17 | 36105117 | HNF 1B | 11 | 94502658 | AMOTL 1 |
| 6 | 10422322 | | 7 | 134832850 | TMEM140 |
| 22 | 50623687 | TRABD | 1 | 1072902 | |
| 14 | 52734525 | PTGDR | 16 | 58498585 | NDRG4 |
| 17 | 76921845 | TIMP2 | 17 | 40825980 | PLEKHH3 |
| | | | 3 | 140660335 | SLC25A36 |

[Step (c)]

The CpG site set 2 for discriminating two kinds among the five kinds of cancers was selected for each combination of two kinds of cancers. Hereinafter, this CpG site set 2 is referred to as a CpG site set 2-5. Specifically, the selection work was carried out for each of the ten kinds of the CpG site sets 2-5 as follows.

All of the CpG sites selected for the CpG site set 1-5 were excluded from the CpG sites constituting the CpG site set P-5, and the remaining CpG sites were used as a population to select CpG sites which had a significant difference in the degree of methylation between the two kinds of cancers and at which primer design was possible. Regarding the primers of the CpG sites, it was checked whether or not DNA amplification could be carried out by single-plex PCR. Regarding the primer that enabled the DNA amplification by single-plex PCR, it was checked whether or not DNA amplification could be carried out by multiplex PCR in one tube. CpG sites for which DNA amplification was confirmed by multiplex PCR were selected for the CpG site set 2-5. Table 2 to Table 11 show the CpG site set 2-5 for each combination of two kinds of cancers.

The positions shown in Table 2 to Table 11 are positions on the coordinates of GRCh37. In a case where the CpG site is known to be included in a transcription region or a transcription regulatory region of a gene, the name of the gene is described in the table.

[Table 2]

| CpG site set 2-5 of colon cancer/pancreatic cancer | | | | | |
|---|---|---|---|---|---|
| Chromosome | Position | Gene | Chromosome | Position | Gene |
| 16 | 31228059 | TRIM72;PYDC 1 | 2 | 74425763 | MTHFD2 |
| 2 | 264164 | ACP1;SH3YL1 | 20 | 4803300 | RASSF2 |
| 1 | 113497999 | SLC16A1 | 11 | 33850605 | |
| 6 | 43142093 | SRF | 19 | 57862627 | ZNF304 |
| 2 | 29338432 | CLIP4 | 1 | 143913409 | FAM72D |
| 7 | 96650192 | DLX5 | 5 | 38557085 | LIFR |
| 14 | 52734529 | PTGDR | 3 | 47029335 | NBEAL2 |
| 19 | 56904901 | ZNF582 | 7 | 134143823 | AKR1B1 |
| 2 | 74425749 | MTHFD2 | 6 | 30652399 | KIAA1949 |
| 1 | 35258594 | GJA4 | 2 | 29338121 | CLIP4 |
| 17 | 36734911 | SRCIN1 | 10 | 135072960 | |
| 1 | 63795934 | | 7 | 27196153 | HOXA7 |
| 8 | 105235606 | RIMS2 | 12 | 6665370 | IFFO1 |
| 1 | 113498106 | SLC16A1 | 19 | 10406145 | ICAM5 |
| 7 | 27205230 | HOXA9 | 19 | 57831678 | ZNF543 |
| 2 | 216877984 | MREG | 18 | 12407806 | SLMO1 |
| 4 | 46391159 | GABRA2 | 4 | 188916726 | ZFP42 |
| 17 | 75370611 | SEPT9 | 12 | 15374303 | RERG |
| 16 | 58497767 | NDRG4 | 13 | 36920660 | SPG20 |
| 2 | 177043255 | | 14 | 24803925 | ADCY4 |
| 14 | 102554977 | HSP90AA 1 | 2 | 242157117 | ANO7 |
| 8 | 72756341 | MSC | 4 | 157997178 | GLRB |
| 20 | 25062447 | VSX1 | 19 | 15090242 | |
| 1 | 151810887 | C2CD4D:LOC100132111 | 16 | 69760928 | NQO1 |
| 5 | 528580 | | 7 | 27195918 | HOXA7 |
| 6 | 27778076 | HIST1H3H | 6 | 108145539 | SCML4 |

[Table 3]

| CpG site set 2-5 of colon cancer/hepatocellular cancer | | | | | |
|---|---|---|---|---|---|
| Chromosome | Position | Gene | Chromosome | Position | Gene |
| 7 | 50344724 | IKZF1 | 6 | 33244976 | B3GALT4 |
| 13 | 24844861 | SPATA13 | 7 | 69064801 | AUTS2 |
| 12 | 53142542 | | 2 | 63283967 | OTX1 |
| 20 | 2781262 | CPXM1 | 2 | 74425593 | MTHFD2 |
| 3 | 142839578 | CHST2 | 1 | 98519504 | |
| 5 | 146258195 | PPP2R2B | 3 | 9993818 | PRRT3 |
| 13 | 28498384 | PDX1 | 2 | 174219336 | CDCA7 |
| 2 | 100938903 | LONRF2 | 19 | 57862638 | ZNF304 |

(continued)

| CpG site set 2-5 of colon cancer/hepatocellular cancer | | | | | |
|---|---|---|---|---|---|
| Chromosome | Position | Gene | Chromosome | Position | Gene |
| 14 | 100438440 | | 6 | 43044771 | PTK7 |
| 7 | 27225528 | HOXA11AS;HOXA11 | 7 | 30722320 | CRHR2 |
| 3 | 142682682 | PAQR9 | 7 | 27194614 | HOXA7 |
| 2 | 66667433 | MEIS1 | 19 | 58220494 | ZNF 154 |
| 4 | 13539099 | | 15 | 72612125 | BRUNOL6 |
| 7 | 94284678 | SGCE;PEG10 | 2 | 264166 | ACP1;SH3YL1 |
| 19 | 3434945 | NFIC | 22 | 38808815 | |
| 7 | 1272515 | UNCX | 15 | 68260574 | |
| 7 | 27205262 | HOXA9 | 5 | 16180076 | MARCH11 |
| 6 | 28303932 | ZNF323 | 3 | 101497982 | FAM55C |
| 9 | 36258600 | GNE | 5 | 112073398 | APC |
| 9 | 27528432 | MOBKL2B | 22 | 32026873 | PISD |
| 17 | 75462189 | SEPT9 | 19 | 43979614 | PHLDB3 |
| 20 | 50248076 | ATP9A | 14 | 105714589 | BTBD6;BRF 1 |
| 4 | 7194519 | SORCS2 | 3 | 9904411 | |
| | | | 6 | 28304088 | ZNF323 |

[Table 4]

| CpG site set 2-5 of colon cancer/bile duct cancer | | | | | |
|---|---|---|---|---|---|
| Chromosome | Position | Gene | Chromosome | Position | Gene |
| 3 | 44803293 | KIF15;KIAA1143 | 20 | 25062447 | VSX1 |
| 1 | 14026584 | PRDM2 | 12 | 28128669 | |
| 20 | 56324074 | | 7 | 42267747 | GLI3 |
| 7 | 21582758 | DNAH11 | 17 | 46806056 | HOXB13 |
| 1 | 247495818 | ZNF496 | 8 | 145013249 | PLEC1 |
| 20 | 19192675 | SLC24A3 | 12 | 24715538 | SOX5 |
| 3 | 50402563 | CACNA2D2 | 7 | 27205224 | HOXA9 |
| 11 | 415111 | SIG1RR | 10 | 102588691 | PAX2 |
| 17 | 35297146 | LHX1 | 7 | 27196296 | HOXA7 |
| 17 | 75369219 | SEPT9 | 10 | 17271994 | VIM |
| 2 | 56151139 | EFEMP 1 | 12 | 25055967 | BCAT1 |
| 10 | 124895441 | HMX3 | 7 | 155167038 | |
| 1 | 33219687 | K1AA1522 | 17 | 38347603 | RAPGEFL1 |
| 19 | 57831909 | ZNF543 | 3 | 181421427 | SOX2OT |
| 6 | 43142093 | SRF | 3 | 9993818 | PRRT3 |
| 7 | 96650192 | DLX5 | 6 | 43044771 | PTK7 |
| 19 | 56904901 | ZNF582 | 1 | 151810589 | LQC100132111;C2CD4D |
| 15 | 45670478 | GATM;LOC145663 | 1 | 143913552 | FAM72D |

(continued)

| CpG site set 2-5 of colon cancer/bile duct cancer | | | | | |
|---|---|---|---|---|---|
| Chromosome | Position | Gene | Chromosome | Position | Gene |
| 12 | 111472375 | CUX2 | 10 | 21463485 | NEBL |
| 1 | 113498106 | SLC16A1 | 3 | 16555379 | RFTN1 |
| 6 | 3023894 | | 12 | 6665370 | IFFO1 |
| 7 | 27205230 | HOXA9 | 12 | 24716204 | SOX5 |
| 2 | 216877984 | MREG | 19 | 57831678 | ZNF543 |
| 11 | 79151188 | ODZ4 | 12 | 15374303 | RERG |
| 5 | 141031122 | FCHSD1 | 2 | 216878510 | MREG |
| 5 | 74231171 | | 19 | 47776728 | PRR24 |

[Table 5]

| CpG site set 2-5 of colon cancer/ovarian cancer | | | | | |
|---|---|---|---|---|---|
| Chromosome | Position | Gene | Chromosome | Position | Gene |
| 13 | 26625307 | SHISA2 | 6 | 10391412 | |
| 16 | 31228059 | TRIM72;PYDC1 | 10 | 11206838 | CUGBP2 |
| 5 | 16180048 | | 7 | 19157193 | TWIST 1 |
| 16 | 23193848 | SCNN1G | 19 | 57862480 | ZNF304 |
| 7 | 96650192 | DLX5 | 14 | 52734325 | PTGDR |
| 2 | 176993632 | HOXD8 | 1 | 145395753 | |
| 6 | 28304249 | ZNF323 | 10 | 17271944 | VIM |
| 5 | 115152494 | CDO1 | 10 | 102588691 | PAX2 |
| 3 | 119041385 | CDGAP | 7 | 27196296 | HOXA7 |
| 3 | 101497980 | FAM55C | 8 | 104383722 | CTHRC1 |
| 1 | 61548783 | NFIA | 5 | 43019873 | |
| 7 | 31375861 | | 3 | 181421427 | SOX2OT |
| 12 | 111472375 | CUX2 | 7 | 100318190 | EPO |
| 10 | 11206870 | CUGBP2 | 12 | 96883381 | |
| 10 | 88730946 | AGAP 11 | 14 | 51027861 | ATL1 |
| 2 | 236579780 | AGAP1 | 2 | 63283967 | OTX1 |
| 10 | 93393030 | PPP1R3C | 2 | 219736312 | WNT6 |
| 11 | 79151188 | ODZ4 | 17 | 42287971 | UBTF |
| 4 | 184644321 | | 10 | 135072960 | |
| 7 | 64349133 | | 7 | 27196153 | HOXA7 |
| 15 | 79382995 | RASGRF1 | 10 | 17270431 | VIM |
| 19 | 57831816 | ZNF543 | 10 | 97802966 | CCNJ |
| 7 | 27196314 | HOXA7 | 3 | 49757016 | AMIGO3;RNF123 |
| 13 | 32605218 | FRY | 4 | 157997178 | GLRB |
| 12 | 28128669 | | 2 | 264204 | ACP1;SH3YL1 |
| 7 | 42267747 | GLI3 | 7 | 27204981 | HOXA9 |

(continued)

| CpG site set 2-5 of colon cancer/ovarian cancer | | | | | |
|---|---|---|---|---|---|
| Chromosome | Position | Gene | Chromosome | Position | Gene |
| 14 | 55596356 | LGALS3 | 3 | 13114797 | IQSEC1 |

[Table 6]

| CpG site set 2-5 of pancreatic cancer/hepatocellular cancer | | | | | |
|---|---|---|---|---|---|
| Chromosome | Position | Gene | Chromosome | Position | Gene |
| 13 | 50070550 | PHF 11 | 4 | 156297858 | MAP9 |
| 14 | 105715025 | BRF 1 ;BTBD6 | 15 | 78556940 | DNAJA4 |
| 1 | 7764737 | CAMTA1 | 4 | 5710372 | EVC2 |
| 2 | 162283705 | | 12 | 111471202 | CUX2 |
| 1 | 46998715 | | 17 | 29297873 | RNF135 |
| 17 | 53343283 | HLF | 1 | 31845959 | FABP3 |
| 12 | 98897187 | | 13 | 107186870 | EFNB2 |
| 12 | 25055262 | BCAT1 | 12 | 25055967 | BCAT1 |
| 4 | 76556042 | CDKL2 | 22 | 38808815 | |
| 5 | 54468707 | MIR449C;CDC20B | 15 | 68260574 | |
| 5 | 178004204 | COL23A1 | 5 | 112073398 | APC |
| 1 | 7692367 | CAMTA1 | 11 | 2905931 | CDKN1C |
| 3 | 141516291 | GRK7 | 14 | 105714589 | BTBD6;BRF 1 |
| 14 | 100259329 | EML 1 | 6 | 152958133 | SYNE1 |
| 3 | 119041529 | CDGAP | 1 | 151812435 | LOC100132111:C2CD4D |
| 15 | 34807221 | | 1 | 183440909 | SMG7 |
| 19 | 11492376 | EPOR | 5 | 102091873 | |
| 4 | 156298050 | MAP9 | 2 | 242973936 | |
| 6 | 28304075 | ZNF323 | 11 | 13030676 | RASSF10 |
| 14 | 105512306 | | 2 | 74782096 | LOXL3;DOK1 |
| 13 | 41187926 | FOXO1 | 10 | 11206868 | CUGBP2 |
| 3 | 40428643 | ENTPD3 | 11 | 70303464 | |
| 17 | 73083812 | SLC16A5 | 7 | 138720803 | ZC3HAV1L |
| 19 | 38755287 | SP1NT2 | 7 | 128470913 | FLNC |
| | | | 6 | 41606439 | MDFI |

[Table 7]

| CpG site set 2-5 of colon cancer/bile duct cancer | | | | | |
|---|---|---|---|---|---|
| Chromosome | Position | Gene | Chromosome | Position | Gene |
| 16 | 31228059 | TRIM72;PYDC 1 | 11 | 70601781 | SHANK2 |
| 9 | 71789653 | TJP2 | 6 | 30139634 | TRIM15 |
| 10 | 119256257 | EMX2OS | 1 | 235116731 | |
| 6 | 39304008 | KIF6 | 1 | 2222253 | SKI |
| 2 | 223176167 | | 19 | 57862612 | ZNF304 |

(continued)

| CpG site set 2-5 of colon cancer/bile duct cancer | | | | | |
|---|---|---|---|---|---|
| Chromosome | Position | Gene | Chromosome | Position | Gene |
| 10 | 71078141 | HK1 | 5 | 16180072 | MARCH11 |
| 12 | 25055262 | BCAT1 | 5 | 115152326 | CDO1 |
| 17 | 75369228 | SEPT9 | 4 | 5710372 | EVC2 |
| 14 | 105940391 | CRIP2 | 3 | 16555379 | RFTN1 |
| 2 | 264164 | ACP1;SH3YL1 | 12 | 24716204 | SOX5 |
| 5 | 141395447 | | 1 | 156893792 | C1orf92 |
| 17 | 4802847 | CHRNE;C17orf107 | 6 | 73332073 | KCNQ5 |
| 2 | 29338432 | CLIP4 | 4 | 159092553 | FAM198B |
| 13 | 41187926 | FOXO 1 | 2 | 219736167 | WNT6 |
| 14 | 52734529 | PTGDR | 8 | 55370336 | SOX17 |
| 19 | 56904901 | ZNF582 | 1 | 3663902 | KIAA0495 |
| 8 | 72756155 | MSC | 19 | 47776728 | PRR24 |
| 5 | 141031125 | FCHSD1 | 19 | 37096148 | ZNF529;ZNF382 |
| 1 | 63795934 | | 16 | 69760928 | NQO1 |
| 11 | 119227105 | USP2 | 1 | 29586418 | PTPRU |
| 5 | 178487382 | ZNF354C | 19 | 30019529 | VSTM2B |
| 2 | 239072674 | | 19 | 38754930 | SPINT2 |
| 19 | 43979464 | PHLDB3 | 6 | 108145539 | SCML4 |

[Table 8]

| CpG site set 2-5 of colon cancer/ovarian cancer | | | | | |
|---|---|---|---|---|---|
| Chromosome | Position | Gene | Chromosome | Position | Gene |
| 16 | 58497395 | NDRG4 | 12 | 111472375 | CUX2 |
| 7 | 28449474 | CREB5 | 2 | 236579780 | AGAP1 |
| 19 | 11450198 | RAB3D | 3 | 193587780 | |
| 16 | 89641296 | CPNE7 | 10 | 106028628 | GSTO2 |
| 7 | 27206544 | HOXA9 | 13 | 32605218 | FRY |
| 1 | 219347458 | LYPLAL1 | 10 | 114136073 | ACSL5 |
| 12 | 111471192 | CUX2 | 6 | 10391412 | |
| 11 | 13690160 | FAR1 | 19 | 57862480 | ZNF304 |
| 2 | 264164 | ACP1;SH3YL1 | 8 | 16884549 | EFHA2 |
| 12 | 24715484 | SOX5 | 14 | 23356059 | REM2 |
| 1 | 119530600 | TBX15 | 14 | 55595768 | LGALS3 |
| 2 | 31456747 | EHD3 | 6 | 33043574 | HLA-DPB1 |
| 1 | 21616641 | ECE1 | 4 | 53728510 | RASL11B |
| 17 | 75368902 | SEPT9 | 12 | 25056243 | BCAT1 |
| 2 | 219736549 | WNT6 | 1 | 151811620 | LOC100132111;C2CD4D |
| 1 | 182584520 | | 17 | 44897431 | WNT3 |

(continued)

| CpG site set 2-5 of colon cancer/ovarian cancer | | | | | |
|---|---|---|---|---|---|
| Chromosome | Position | Gene | Chromosome | Position | Gene |
| 16 | 23193848 | SCNN1G | 2 | 219736167 | WNT6 |
| 2 | 74426422 | MTHFD2 | 20 | 37434552 | PPP1R16B |
| 1 | 236558465 | EDARADD | 8 | 38411708 | |
| 2 | 66666470 | MEIS1 | 2 | 31456964 | EHD3 |
| 12 | 54088972 | | 15 | 45670865 | LOC145663;GATM |
| 6 | 28304249 | ZNF323 | 11 | 13690157 | FAR1 |
| 1 | 154297848 | ATP8B2 | 2 | 131721099 | ARHGEF4 |
| 4 | 76555777 | CDKL2 | 12 | 111618936 | CUX2 |
| 7 | 27196555 | HOXA7 | 1 | 29586418 | PTPRU |
| 12 | 25102072 | BCAT1 | 5 | 40681893 | PTGER4 |
| 11 | 128564874 | FLI1 | 3 | 13114797 | IQSEC1 |
| 10 | 11207907 | CUGBP2 | 1 | 38513245 | POU3F1 |

[Table 9]

| CpG | | site set 2-5 of hepatocellular cancer/bile duct cancer | | | |
|---|---|---|---|---|---|
| Chromosome | Position | Gene | Chromosome | Position | Gene |
| 14 | 105940179 | CR1P2 | 17 | 75369055 | SEPT9 |
| 17 | 53343283 | HLF | 14 | 36003826 | INSM2 |
| 12 | 98897187 | | 15 | 78556940 | DNAJA4 |
| 4 | 76556042 | CDKL2 | 12 | 111471202 | CUX2 |
| 5 | 54468707 | MIR449C;CDC20B | 8 | 145013249 | PLEC1 |
| 19 | 56904945 | ZNF582 | 19 | 3435350 | NFIC |
| 7 | 50348174 | IKZF1 | 2 | 131130103 | PTPN18 |
| 11 | 507455 | RNH1 | 2 | 127414108 | GYPC |
| 11 | 14927004 | | 1 | 12123262 | TNFRSF8 |
| 6 | 73331680 | KCNQ5 | 7 | 27205159 | HOXA9 |
| 22 | 44577135 | PARVG | 11 | 830320 | EFCAB4A |
| 7 | 100488942 | ACHE | 8 | 67874178 | |
| 14 | 100259329 | EML1 | 2 | 264166 | ACP1;SH3YL1 |
| 3 | 119041529 | CDGAP | 22 | 38808815 | |
| 6 | 42739021 | | 5 | 112073398 | APC |
| 4 | 126237421 | FAT 4 | 9 | 36258171 | GNE |
| 3 | 119041385 | CDGAP | 11 | 2905931 | CDKN1C |
| 2 | 239072674 | | 19 | 43979614 | PHLDB3 |
| 7 | 157361692 | PTPRN2 | 1 | 156405438 | |
| 6 | 3023894 | | 12 | 25056243 | BCAT1 |
| 11 | 79151188 | ODZ4 | 1 | 151811620 | LOC100132111;C2CD4D |
| 3 | 40428643 | ENTPD3 | 4 | 76555634 | CDKL 2 |

(continued)

| CpG | | site set 2-5 of hepatocellular cancer/bile duct cancer | | | |
|---|---|---|---|---|---|
| Chromosome | Position | Gene | Chromosome | Position | Gene |
| 7 | 138720786 | ZC3HAV1L | 6 | 42739049 | |
| 2 | 100938813 | LONRF2 | 12 | 133065941 | FBRSL 1 |
| 19 | 38755287 | SPINT2 | 10 | 114134915 | ACSL5 |
| 4 | 156297858 | MAP9 | 2 | 242973936 | |
| | | | 2 | 119606746 | EN1 |

[Table 10]

| CpG site set 2-5 of hepatocellular cancer/ovarian cancer | | | | | |
|---|---|---|---|---|---|
| Chromosome | Position | Gene | Chromosome | Position | Gene |
| 5 | 54468707 | MIR449C;CDC20B | 13 | 32605218 | FRY |
| 19 | 56904945 | ZNF582 | 1 | 145395753 | |
| 7 | 50348174 | IKZF1 | 5 | 177540239 | N4BP3 |
| 14 | 105940391 | CRIP2 | 1 | 31845959 | FABP3 |
| 6 | 73331680 | KCNQ5 | 8 | 16884549 | EFHA2 |
| 17 | 4802847 | CHRNE;C17orf107 | 2 | 131130103 | PTPN18 |
| 4 | 156298050 | MAP9 | 5 | 162931471 | MAT2B |
| 16 | 27437900 | IL21R | 12 | 25055967 | BCAT1 |
| 16 | 23193848 | SCNN1G | 8 | 104383722 | CTHRC1 |
| 14 | 105512306 | | 8 | 67874178 | |
| 2 | 74426422 | MTHFD2 | 15 | 60296981 | FOXB1 |
| 5 | 141031147 | FCHSD1 | 7 | 27205658 | HOXA9 |
| 7 | 27196320 | HOXA7 | 9 | 36258171 | GNE |
| 1 | 236558465 | EDARADD | 13 | 32605406 | FRY |
| 1 | 154297848 | ATP8B2 | 1 | 43390705 | |
| 11 | 119227105 | USP2 | 8 | 27183097 | PTK2B |
| 12 | 25102072 | BCAT 1 | 3 | 16555379 | RFTN1 |
| 22 | 50623692 | TRABD | 10 | 17270431 | VIM |
| 3 | 101497980 | FAM55C | 4 | 76555634 | CDKL2 |
| 1 | 156051324 | MEX3A | 6 | 42739049 | |
| 10 | 11207907 | CUGBP2 | 12 | 133065941 | FBRSL 1 |
| 19 | 43979464 | PHLDB3 | 19 | 47776728 | PRR24 |
| 10 | 11206813 | CUGBP2 | 5 | 176831364 | F12 |
| 7 | 27195602 | HOXA7 | 7 | 27204981 | HOXA9 |
| 3 | 193587780 | | 16 | 46783018 | MYLK3 |
| 10 | 106028628 | GSTO2 | 1 | 29586418 | PTPRU |
| 3 | 40428643 | ENTPD3 | 16 | 85932666 | IRF8 |
| 5 | 16180072 | MARCH 11 | 19 | 38754930 | SPINT2 |
| | | | 6 | 108145539 | SCML4 |

[Table 11]

| CpG site set 2-5 of bile duct cancer/ovarian cancer | | | | | |
|---|---|---|---|---|---|
| Chromosome | Position | Gene | Chromosome | Position | Gene |
| 14 | 64805784 | ESR2 | 13 | 88324597 | SLITRK5 |
| 9 | 71789653 | TJP2 | 1 | 236558465 | EDARADD |
| 16 | 58498151 | NDRG4 | 14 | 52734529 | PTGDR |
| 7 | 73624319 | LAT2 | 12 | 54088972 | |
| 19 | 30016147 | VSTM2B | 15 | 72612720 | BRUNOL6 |
| 11 | 44590889 | CD82 | 6 | 28304249 | ZNF323 |
| 6 | 39304008 | KIF6 | 1 | 154297848 | ATP8B2 |
| 10 | 71078141 | HK1 | 4 | 76555777 | CDKL2 |
| 7 | 27206544 | HOXA9 | 7 | 27196555 | HOXA7 |
| 12 | 124246919 | DNAH10 | 12 | 25102072 | BCAT1 |
| 17 | 75369228 | SEPT9 | 19 | 43979464 | PHLDB3 |
| 6 | 150358985 | | 6 | 30140018 | TRIM15 |
| 1 | 219347458 | LYPLAL1 | 14 | 60618622 | DHRS7 |
| 12 | 111471192 | CUX2 | 15 | 83953880 | BNC1 |
| 19 | 1467952 | APC2 | 14 | 52734325 | PTGDR |
| 11 | 13690702 | FAR1 | 1 | 145395753 | |
| 13 | 36920813 | SPG20 | 14 | 23356059 | REM2 |
| 1 | 24645917 | GRHL3 | 5 | 162931471 | MAT2B |
| 2 | 31456747 | EHD3 | 3 | 16555379 | RFTN1 |
| 1 | 182584520 | | 10 | 17270431 | VIM |
| 17 | 4802847 | CHRNE;C17orf107 | 1 | 151811620 | LOC100132111;C2CD4D |
| 6 | 130339617 | L3MBTL3 | 19 | 47776728 | PRR24 |
| 16 | 23193848 | SCNN1G | 1 | 29586418 | PTPRU |
| | | | 1 | 38513245 | POU3F1 |

[Step (d): Production of primer mix for primary examination]

A primer for each of 61 CpG sites constituting the CpG site set 1-5 was synthesized. An equal amount of 100 μM of each of the primers was mixed in a single tube, and the mixed solution was diluted to 1 μM to prepare a primer mix. Each primer for each CpG site is the primer designed in the step (b).

[Step (e): Production of primer mix for secondary examination]

A primer for each of the CpG sites constituting the CpG site set was synthesized for each of ten kinds of the CpG site sets 2-5. For each of ten kinds of the CpG site sets 2-5, an equal amount of 100 μM of each of the primers was mixed in a single tube, and the mixed solution was diluted to 1 μM to prepare a primer mix. Each primer for each CpG site is the primer designed in the step (c).

<Example 2: Cancer examination>

[Creation of classifier for primary examination]

The estimation of the kind of cancer was carried out using a random forest (RF) and a support vector machine (SVM), which are machine learning models.

The data from the 937 specimens used for the extraction of the CpG site set P-5 was read into the machine learning model as training data, and Orange (https://orangedatamining.com/), which is a data mining software based on the open source, was used to create an RF classifier and an SVM classifier. The created classifiers were evaluated by Orange, and it was confirmed that each of the classifiers has a classification accuracy (CA) of 0.95 or more.

[Creation of classifier for secondary examination]

An RF classifier and an SVM classifier were created for each combination of the two kinds of cancers in the same manner as in the creation of the classifier for the primary examination, where data from 200 or more specimens was used as training data for each combination of two kinds selected from the five kinds of cancers. The created classifiers were evaluated by Orange, and it was confirmed that each of the classifiers has a CA of 0.95 or more.

[DNA extraction]

Blood plasma was obtained from each of two patients with colon cancer. They are referred to as a sample A and a sample B, respectively.
The extraction of cfDNA from the blood plasma was carried out using a QIAamp Circulating Nucleic Acid Kit (manufactured by QIAGEN N.V.) as follows according to the standard protocol.
To a 50 mL centrifuge tube, 200 μL of QIAGEN Proteinase K, 2 mL of blood plasma, and 1.6 mL of Buffer ACL were sequentially added, and the lid was closed, followed by mixing with pulse vortexing for 30 seconds. Next, incubation was carried out at a temperature of 60°C for 30 minutes. Next, 3.6 mL of Buffer ACB was added thereto, and the lid was closed, followed by mixing with pulse vortexing for 15 to 30 seconds. Next, incubation was carried out on ice for 5 minutes. Next, DNA was washed using a manifold QIAvac 24 Plus for suction treatment of a spin column, a connector VacConnector, and a spin column QIAamp Mini Column, and the DNA was recovered with a 20 μL eluent (manufactured by Buffer AVE).

[Bisulfite treatment]

The obtained DNA was treated with an EZ DNA Methylation Gold Kit (manufactured by Zymo research Corporation) according to the standard protocol of the product.

[Primary examination]

Using the primer mix for primary examination described above, the DNA after the bisulfite treatment was amplified by multiplex PCR. Multiplex PCR was carried out using KOD -Multi & Epi- (manufactured by TOYOBO Co., Ltd.) according to the instruction manual of this product. 25 μL of 2X PCR Buffer for KOD -Multi & Epi-, 1 μL of KOD -Multi & Epi-, 15 μL of 1 μM primer mix, 8.5 μL of the DNA after the bisulfite treatment, and 0.5 μL of water was dispensed into a PCR tube. Regarding PCR, one cycle of 94°C/2 minutes was carried out, and then three steps of 98°C/10 seconds, 58°C/30 seconds, and 68°C/15 seconds were repeated by 40 cycles. The amplification reaction solution was purified using AMPure XP (manufactured by Beckman Coulter, Inc.), and the purified DNA was recovered in 40 μL of a Tris-EDTA buffer solution.
Using the recovered DNA and a general primer used for Index Addition PCR, DNA was amplified by Index Addition PCR. Index addition PCR was carried out using a Multiplex PCR Assay Kit (manufactured by Takara Bio Inc.). 1 μL of each of 1.25 μM primers, 0.125 μL of Multiplex PCR Mix 1, 12.5 μL of Multiplex PCR Mix 2, were used, and the final liquid volume was adjusted to 25 μL of water to prepare a reaction solution. Regarding PCR, one cycle of 94°C/3 minutes was carried out, and then three steps of 94°C/45 seconds, 50°C/60 seconds, and 72°C/30 seconds were repeated by 5 cycles, and three steps of 94°C/45 seconds, 55°C/60 seconds, and 72°C/30 seconds were repeated by 11 cycles.
The obtained PCR product was purified using an AMPure XP Kit (manufactured by Beckman Coulter, Inc.). The concentration of the purified DNA was quantified using BioAnalyzer (manufactured by Agilent Technologies, Inc.) and more accurately quantified using a KAPA Library Quantification Kit (manufactured by KAPA Biosystems, Inc.). The purified DNA was used as a specimen and sequenced using a Miseq Reagent Kit v2 300 Cycle (manufactured by Illumina, Inc.). The information on the degree of methylation of 61 CpG sites was acquired by mapping the obtained FastQ file to a human genome sequence using Bismark.

[Estimation of kind of cancer with primary examination classifier]

In the estimation of the kind of cancer with the classifier for the primary examination, the cancer having the highest degree of certainty was output as "estimated first place", and the cancer having the second highest degree of certainty was output as "estimated second place".

The information on the degree of methylation of each of the sample A and the sample B was evaluated with the RF classifier and the SVM classifier for the primary examination. The results are shown in Table 12. The numerical values in Table 12 are contribution ratios (which indicate numerical values between 0 and 1) output by the data mining software Orange, which are used as the degree of certainty. A degree of certainty of 0.5 was used as a threshold value.

[Table 12]

| | Sample A | | Sample B | |
|---|---|---|---|---|
| | RF | SVM | RF | SVM |
| Estimated first place | Colon cancer 1.0 | Colon cancer 0.73 | Colon cancer 0.36 | Colon cancer 0.91 |
| Estimated second place | Absent | Lung cancer 0.11 | Hepatocellular cancer 0.20 | Hepatocellular cancer 0.02 |
| RF: random forest, SVM: support vector machine | | | | |

- Sample A -

The estimated first place with both the RF classifier and the SVM classifier was colon cancer, and the degree of certainty of the estimated first place with both the RF classifier and the SVM classifier was 0.5 or more. It was determined that the secondary examination of the sample A was unnecessary, and the sample A was determined to have colon cancer only by the primary examination.

- Sample B -

The degree of certainty of the estimated first place with the RF classifier was less than 0.5, and the difference in the degree of certainty between the estimated first place and the estimated second place with the RF classifier was small. It was determined that the secondary examination for discriminating the colon cancer from the hepatocellular cancer was necessary for the sample B.

[Secondary examination]

Among the primer mixes for secondary examinations described above, a primer mix matching with the CpG site set 2-5 (that is, the CpG site set 2-5 shown in Table 3) for discriminating colon cancer from hepatocellular cancer was used. Using this primer mix, the information on the degree of methylation of 47 CpG sites was acquired in the same manner as in the primary examination.

[Estimation of kind of cancer with secondary examination classifier]

The information on the degree of methylation of the sample B was evaluated with a classifier for the secondary examination where the classifier was for discriminating colon cancer from hepatocellular cancer. The results are shown in Table 13. The numerical values in Table 13 are contribution ratios (which indicate numerical values between 0 and 1) output by the data mining software Orange, which are used as the degree of certainty. A degree of certainty of 0.5 was used as a threshold value.

[Table 13]

| | Sample B | |
|---|---|---|
| | RF | SVM |
| Estimated first place | Colon cancer 0.77 | Colon cancer 0.97 |
| Estimated second place | Hepatocellular cancer 0.23 | Hepatocellular cancer 0.03 |
| RF: random forest, SVM: support vector machine | | |

The estimated first place with both the RF classifier and the SVM classifier was colon cancer, and the degree of certainty of the estimated first place with both the RF classifier and the SVM classifier was 0.5 or more. The sample B was

determined to have colon cancer.

In Example for the sample B, the reliability degree of the first DNA methylation analysis was not high; however, the second DNA methylation analysis was carried out based on the results of the first analysis, whereby finally, it was possible to make a determination with a high reliability degree.

## Claims

**1.** A cancer examination reagent set comprising:

the following primary examination reagent; and
the following secondary examination reagent,
the primary examination reagent: primers specifically designed for analyzing a degree of methylation of each of all CpG sites constituting a CpG site set 1 selected for examining n kinds of cancers, where n is a natural number of 3 or more, wherein the CpG site set 1 is shown in Table 1, wherein the positions are the positions on the coordinates of Genome Reference Consortium Human Build 37:

Table 1

| CpG site set 1 | | | |
|---|---|---|---|
| Chromosome | Position | Chromosome | Position |
| 21 | 36399258 | 1 | 14026590 |
| 10 | 97802941 | 2 | 37899953 |
| 7 | 73508618 | 7 | 27196302 |
| 8 | 55370423 | 2 | 144695078 |
| 19 | 57831909 | 10 | 122708532 |
| 16 | 67034882 | 10 | 113943887 |
| 16 | 58498190 | 7 | 96634660 |
| 5 | 169724656 | 9 | 124461171 |
| 12 | 117798954 | 14 | 23356251 |
| 12 | 105478345 | 7 | 27196365 |
| 1 | 78511856 | 5 | 43007632 |
| 3 | 142682652 | 9 | 130212635 |
| 11 | 67351271 | 7 | 16794078 |
| 1 | 29586414 | 14 | 37126902 |
| 1 | 143913847 | 6 | 152623304 |
| 17 | 61524073 | 1 | 63795711 |
| 15 | 90208810 | 5 | 40681137 |
| 1 | 151810893 | 10 | 102792249 |
| 1 | 29586299 | 5 | 178004204 |
| 6 | 10887047 | 3 | 168864101 |
| 12 | 104850745 | 7 | 42267719 |
| 13 | 98628060 | 16 | 215960 |
| 15 | 76633086 | 7 | 142986693 |
| 7 | 1272545 | 2 | 162280519 |
| 17 | 75369939 | 11 | 66624841 |
| 17 | 36105117 | 11 | 94502658 |

(continued)

| CpG site set 1 | | | |
|---|---|---|---|
| Chromosome | Position | Chromosome | Position |
| 6 | 10422322 | 7 | 134832850 |
| 22 | 50623687 | 1 | 1072902 |
| 14 | 52734525 | 16 | 58498585 |
| 17 | 76921845 | 17 | 40825980 |
| | | 3 | 140660335 |

the secondary examination reagent: a set of $_nC_2$ kinds of primers specifically designed for analyzing a degree of methylation of each of all CpG sites constituting $_nC_2$ kinds of CpG site sets 2, the $_nC_2$ kinds being selected for respectively discriminating two kinds among the n kinds of cancers, wherein the CpG site sets 2 are shown in the following Tables 2 to 11, wherein the positions are the positions on the coordinates of Genome Reference Consortium Human Build 37:

Table 2

| CpG site set 2 of colon cancer/pancreatic cancer | | | |
|---|---|---|---|
| Chromosome | Position | Chromosome | Position |
| 16 | 31228059 | 2 | 74425763 |
| 2 | 264164 | 20 | 4803300 |
| 1 | 113497999 | 11 | 33850605 |
| 6 | 43142093 | 19 | 57862627 |
| 2 | 29338432 | 1 | 143913409 |
| 7 | 96650192 | 5 | 38557085 |
| 14 | 52734529 | 3 | 47029335 |
| 19 | 56904901 | 7 | 134143823 |
| 2 | 74425749 | 6 | 30652399 |
| 1 | 35258594 | 2 | 29338121 |
| 17 | 36734911 | 10 | 135072960 |
| 1 | 63795934 | 7 | 27196153 |
| 8 | 105235606 | 12 | 6665370 |
| 1 | 113498106 | 19 | 10406145 |
| 7 | 27205230 | 19 | 57831678 |
| 2 | 216877984 | 18 | 12407806 |
| 4 | 46391159 | 4 | 188916726 |
| 17 | 75370611 | 12 | 15374303 |
| 16 | 58497767 | 13 | 36920660 |
| 2 | 177043255 | 14 | 24803925 |
| 14 | 102554977 | 2 | 242157117 |
| 8 | 72756341 | 4 | 157997178 |
| 20 | 25062447 | 19 | 15090242 |
| 1 | 151810887 | 16 | 69760928 |
| 5 | 528580 | 7 | 27195918 |

(continued)

| CpG site set 2 of colon cancer/pancreatic cancer | | | |
|---|---|---|---|
| Chromosome | Position | Chromosome | Position |
| 6 | 27778076 | 6 | 108145539 |

Table 3

| CpG site set 2 of colon cancer/hepatocellular cancer | | | |
|---|---|---|---|
| Chromosome | Position | Chromosome | Position |
| 7 | 50344724 | 6 | 33244976 |
| 13 | 24844861 | 7 | 69064801 |
| 12 | 53142542 | 2 | 63283967 |
| 20 | 2781262 | 2 | 74425593 |
| 3 | 142839578 | 1 | 98519504 |
| 5 | 146258195 | 3 | 9993818 |
| 13 | 28498384 | 2 | 174219336 |
| 2 | 100938903 | 19 | 57862638 |
| 14 | 100438440 | 6 | 43044771 |
| 7 | 27225528 | 7 | 30722320 |
| 3 | 142682682 | 7 | 27194614 |
| 2 | 66667433 | 19 | 58220494 |
| 4 | 13539099 | 15 | 72612125 |
| 7 | 94284678 | 2 | 264166 |
| 19 | 3434945 | 22 | 38808815 |
| 7 | 1272515 | 15 | 68260574 |
| 7 | 27205262 | 5 | 16180076 |
| 6 | 28303932 | 3 | 101497982 |
| 9 | 36258600 | 5 | 112073398 |
| 9 | 27528432 | 22 | 32026873 |
| 17 | 75462189 | 19 | 43979614 |
| 20 | 50248076 | 14 | 105714589 |
| 4 | 7194519 | 3 | 9904411 |
| | | 6 | 28304088 |

Table 4

| CpG site set 2 of colon cancer/bile duct cancer | | | |
|---|---|---|---|
| Chromosome | Position | Chromosome | Position |
| 3 | 44803293 | 20 | 25062447 |
| 1 | 14026584 | 12 | 28128669 |
| 20 | 56324074 | 7 | 42267747 |
| 7 | 21582758 | 17 | 46806056 |
| 1 | 247495818 | 8 | 145013249 |
| 20 | 19192675 | 12 | 24715538 |

(continued)

| CpG site set 2 of colon cancer/bile duct cancer | | | |
|---|---|---|---|
| Chromosome | Position | Chromosome | Position |
| 3 | 50402563 | 7 | 27205224 |
| 11 | 415111 | 10 | 102588691 |
| 17 | 35297146 | 7 | 27196296 |
| 17 | 75369219 | 10 | 17271994 |
| 2 | 56151139 | 12 | 25055967 |
| 10 | 124895441 | 7 | 155167038 |
| 1 | 33219687 | 17 | 38347603 |
| 19 | 57831909 | 3 | 181421427 |
| 6 | 43142093 | 3 | 9993818 |
| 7 | 96650192 | 6 | 43044771 |
| 19 | 56904901 | 1 | 151810589 |
| 15 | 45670478 | 1 | 143913552 |
| 12 | 111472375 | 10 | 21463485 |
| 1 | 113498106 | 3 | 16555379 |
| 6 | 3023894 | 12 | 6665370 |
| 7 | 27205230 | 12 | 24716204 |
| 2 | 216877984 | 19 | 57831678 |
| 11 | 79151188 | 12 | 15374303 |
| 5 | 141031122 | 2 | 216878510 |
| 5 | 74231171 | 19 | 47776728 |

Table 5

| CpG site set 2 of colon cancer/ovarian cancer | | | |
|---|---|---|---|
| Chromosome | Position | Chromosome | Position |
| 13 | 26625307 | 6 | 10391412 |
| 16 | 31228059 | 10 | 11206838 |
| 5 | 16180048 | 7 | 19157193 |
| 16 | 23193848 | 19 | 57862480 |
| 7 | 96650192 | 14 | 52734325 |
| 2 | 176993632 | 1 | 145395753 |
| 6 | 28304249 | 10 | 17271944 |
| 5 | 115152494 | 10 | 102588691 |
| 3 | 119041385 | 7 | 27196296 |
| 3 | 101497980 | 8 | 104383722 |
| 1 | 61548783 | 5 | 43019873 |
| 7 | 31375861 | 3 | 181421427 |
| 12 | 111472375 | 7 | 100318190 |
| 10 | 11206870 | 12 | 96883381 |

(continued)

| CpG site set 2 of colon cancer/ovarian cancer | | | |
|---|---|---|---|
| Chromosome | Position | Chromosome | Position |
| 10 | 88730946 | 14 | 51027861 |
| 2 | 236579780 | 2 | 63283967 |
| 10 | 93393030 | 2 | 219736312 |
| 11 | 79151188 | 17 | 42287971 |
| 4 | 184644321 | 10 | 135072960 |
| 7 | 64349133 | 7 | 27196153 |
| 15 | 79382995 | 10 | 17270431 |
| 19 | 57831816 | 10 | 97802966 |
| 7 | 27196314 | 3 | 49757016 |
| 13 | 32605218 | 4 | 157997178 |
| 12 | 28128669 | 2 | 264204 |
| 7 | 42267747 | 7 | 27204981 |
| 14 | 55596356 | 3 | 13114797 |

Table 6

| CpG site set 2 of pancreatic cancer/hepatocellular cancer | | | |
|---|---|---|---|
| Chromosome | Position | Chromosome | Position |
| 13 | 50070550 | 4 | 156297858 |
| 14 | 105715025 | 15 | 78556940 |
| 1 | 7764737 | 4 | 5710372 |
| 2 | 162283705 | 12 | 111471202 |
| 1 | 46998715 | 17 | 29297873 |
| 17 | 53343283 | 1 | 31845959 |
| 12 | 98897187 | 13 | 107186870 |
| 12 | 25055262 | 12 | 25055967 |
| 4 | 76556042 | 22 | 38808815 |
| 5 | 54468707 | 15 | 68260574 |
| 5 | 178004204 | 5 | 112073398 |
| 1 | 7692367 | 11 | 2905931 |
| 3 | 141516291 | 14 | 105714589 |
| 14 | 100259329 | 6 | 152958133 |
| 3 | 119041529 | 1 | 151812435 |
| 15 | 34807221 | 1 | 183440909 |
| 19 | 11492376 | 5 | 102091873 |
| 4 | 156298050 | 2 | 242973936 |
| 6 | 28304075 | 11 | 13030676 |
| 14 | 105512306 | 2 | 74782096 |
| 13 | 41187926 | 10 | 11206868 |

(continued)

| CpG site set 2 of pancreatic cancer/hepatocellular cancer | | | |
|---|---|---|---|
| Chromosome | Position | Chromosome | Position |
| 3 | 40428643 | 11 | 70303464 |
| 17 | 73083812 | 7 | 138720803 |
| 19 | 38755287 | 7 | 128470913 |
| | | 6 | 41606439 |

Table 7

| CpG site set 2 of colon cancer/bile duct cancer | | | |
|---|---|---|---|
| Chromosome | Position | Chromosome | Position |
| 16 | 31228059 | 11 | 70601781 |
| 9 | 71789653 | 6 | 30139634 |
| 10 | 119256257 | 1 | 235116731 |
| 6 | 39304008 | 1 | 2222253 |
| 2 | 223176167 | 19 | 57862612 |
| 10 | 71078141 | 5 | 16180072 |
| 12 | 25055262 | 5 | 115152326 |
| 17 | 75369228 | 4 | 5710372 |
| 14 | 105940391 | 3 | 16555379 |
| 2 | 264164 | 12 | 24716204 |
| 5 | 141395447 | 1 | 156893792 |
| 17 | 4802847 | 6 | 73332073 |
| 2 | 29338432 | 4 | 159092553 |
| 13 | 41187926 | 2 | 219736167 |
| 14 | 52734529 | 8 | 55370336 |
| 19 | 56904901 | 1 | 3663902 |
| 8 | 72756155 | 19 | 47776728 |
| 5 | 141031125 | 19 | 37096148 |
| 1 | 63795934 | 16 | 69760928 |
| 11 | 119227105 | 1 | 29586418 |
| 5 | 178487382 | 19 | 30019529 |
| 2 | 239072674 | 19 | 38754930 |
| 19 | 43979464 | 6 | 108145539 |

Table 8

| CpG site set 2 of colon cancer/ovarian cancer | | | |
|---|---|---|---|
| Chromosome | Position | Chromosome | Position |
| 16 | 58497395 | 12 | 111472375 |
| 7 | 28449474 | 2 | 236579780 |
| 19 | 11450198 | 3 | 193587780 |
| 16 | 89641296 | 10 | 106028628 |

(continued)

| CpG site set 2 of colon cancer/ovarian cancer | | | |
|---|---|---|---|
| Chromosome | Position | Chromosome | Position |
| 7 | 27206544 | 13 | 32605218 |
| 1 | 219347458 | 10 | 114136073 |
| 12 | 111471192 | 6 | 10391412 |
| 11 | 13690160 | 19 | 57862480 |
| 2 | 264164 | 8 | 16884549 |
| 12 | 24715484 | 14 | 23356059 |
| 1 | 119530600 | 14 | 55595768 |
| 2 | 31456747 | 6 | 33043574 |
| 1 | 21616641 | 4 | 53728510 |
| 17 | 75368902 | 12 | 25056243 |
| 2 | 219736549 | 1 | 151811620 |
| 1 | 182584520 | 17 | 44897431 |
| 16 | 23193848 | 2 | 219736167 |
| 2 | 74426422 | 20 | 37434552 |
| 1 | 236558465 | 8 | 38411708 |
| 2 | 66666470 | 2 | 31456964 |
| 12 | 54088972 | 15 | 45670865 |
| 6 | 28304249 | 11 | 13690157 |
| 1 | 154297848 | 2 | 131721099 |
| 4 | 76555777 | 12 | 111618936 |
| 7 | 27196555 | 1 | 29586418 |
| 12 | 25102072 | 5 | 40681893 |
| 11 | 128564874 | 3 | 13114797 |
| 10 | 11207907 | 1 | 38513245 |

Table 9

| CpG site set 2 of hepatocellular cancer/bile duct cancer | | | |
|---|---|---|---|
| Chromosome | Position | Chromosome | Position |
| 14 | 105940179 | 17 | 75369055 |
| 17 | 53343283 | 14 | 36003826 |
| 12 | 98897187 | 15 | 78556940 |
| 4 | 76556042 | 12 | 111471202 |
| 5 | 54468707 | 8 | 145013249 |
| 19 | 56904945 | 19 | 3435350 |
| 7 | 50348174 | 2 | 131130103 |
| 11 | 507455 | 2 | 127414108 |
| 11 | 14927004 | 1 | 12123262 |
| 6 | 73331680 | 7 | 27205159 |

(continued)

| CpG site set 2 of hepatocellular cancer/bile duct cancer | | | |
|---|---|---|---|
| Chromosome | Position | Chromosome | Position |
| 22 | 44577135 | 11 | 830320 |
| 7 | 100488942 | 8 | 67874178 |
| 14 | 100259329 | 2 | 264166 |
| 3 | 119041529 | 22 | 38808815 |
| 6 | 42739021 | 5 | 112073398 |
| 4 | 126237421 | 9 | 36258171 |
| 3 | 119041385 | 11 | 2905931 |
| 2 | 239072674 | 19 | 43979614 |
| 7 | 157361692 | 1 | 156405438 |
| 6 | 3023894 | 12 | 25056243 |
| 11 | 79151188 | 1 | 151811620 |
| 3 | 40428643 | 4 | 76555634 |
| 7 | 138720786 | 6 | 42739049 |
| 2 | 100938813 | 12 | 133065941 |
| 19 | 38755287 | 10 | 114134915 |
| 4 | 156297858 | 2 | 242973936 |
| | | 2 | 119606746 |

Table 10

| CpG site set 2 of hepatocellular cancer/ovarian cancer | | | |
|---|---|---|---|
| Chromosome | Position | Chromosome | Position |
| 5 | 54468707 | 13 | 32605218 |
| 19 | 56904945 | 1 | 145395753 |
| 7 | 50348174 | 5 | 177540239 |
| 14 | 105940391 | 1 | 31845959 |
| 6 | 73331680 | 8 | 16884549 |
| 17 | 4802847 | 2 | 131130103 |
| 4 | 156298050 | 5 | 162931471 |
| 16 | 27437900 | 12 | 25055967 |
| 16 | 23193848 | 8 | 104383722 |
| 14 | 105512306 | 8 | 67874178 |
| 2 | 74426422 | 15 | 60296981 |
| 5 | 141031147 | 7 | 27205658 |
| 7 | 27196320 | 9 | 36258171 |
| 1 | 236558465 | 13 | 32605406 |
| 1 | 154297848 | 1 | 43390705 |
| 11 | 119227105 | 8 | 27183097 |
| 12 | 25102072 | 3 | 16555379 |

(continued)

| CpG site set 2 of hepatocellular cancer/ovarian cancer | | | |
|---|---|---|---|
| Chromosome | Position | Chromosome | Position |
| 22 | 50623692 | 10 | 17270431 |
| 3 | 101497980 | 4 | 76555634 |
| 1 | 156051324 | 6 | 42739049 |
| 10 | 11207907 | 12 | 133065941 |
| 19 | 43979464 | 19 | 47776728 |
| 10 | 11206813 | 5 | 176831364 |
| 7 | 27195602 | 7 | 27204981 |
| 3 | 193587780 | 16 | 46783018 |
| 10 | 106028628 | 1 | 29586418 |
| 3 | 40428643 | 16 | 85932666 |
| 5 | 16180072 | 19 | 38754930 |
| | | 6 | 108145539 |

Table 11

| CpG site set 2 of bile duct cancer/ovarian cancer | | | |
|---|---|---|---|
| Chromosome | Position | Chromosome | Position |
| 14 | 64805784 | 13 | 88324597 |
| 9 | 71789653 | 1 | 236558465 |
| 16 | 58498151 | 14 | 52734529 |
| 7 | 73624319 | 12 | 54088972 |
| 19 | 30016147 | 15 | 72612720 |
| 11 | 44590889 | 6 | 28304249 |
| 6 | 39304008 | 1 | 154297848 |
| 10 | 71078141 | 4 | 76555777 |
| 7 | 27206544 | 7 | 27196555 |
| 12 | 124246919 | 12 | 25102072 |
| 17 | 75369228 | 19 | 43979464 |
| 6 | 150358985 | 6 | 30140018 |
| 1 | 219347458 | 14 | 60618622 |
| 12 | 111471192 | 15 | 83953880 |
| 19 | 1467952 | 14 | 52734325 |
| 11 | 13690702 | 1 | 145395753 |
| 13 | 36920813 | 14 | 23356059 |
| 1 | 24645917 | 5 | 162931471 |
| 2 | 31456747 | 3 | 16555379 |
| 1 | 182584520 | 10 | 17270431 |
| 17 | 4802847 | 1 | 151811620 |
| 6 | 130339617 | 19 | 47776728 |

(continued)

| CpG site set 2 of bile duct cancer/ovarian cancer | | | |
|---|---|---|---|
| Chromosome | Position | Chromosome | Position |
| 16 | 23193848 | 1 | 29586418 |
| | | 1 | 38513245 |

2. A production method for a cancer examination reagent set including a primary examination reagent and a secondary examination reagent, the production method for a cancer examination reagent set, comprising the following (a) to (e):

(a) creating a CpG site set P consisting of CpG sites having a significant difference in degree of methylation in at least one kind among n kinds of cancers, where n is a natural number of 3 or more;
(b) selecting a part of the CpG sites constituting the CpG site set P to create a CpG site set 1;
(c) selecting a part of the CpG sites constituting the CpG site set P to create each of ${}_nC_2$ kinds of CpG site sets 2 for discriminating two kinds among the n kinds of cancers;
(d) producing primers specifically designed for analyzing a degree of methylation of the CpG site set 1 to form the primary examination reagent; and
(e) producing ${}_nC_2$ kinds of primers specifically designed for analyzing degrees of methylation of the ${}_nC_2$ kinds of CpG site sets 2, respectively, to form the secondary examination reagent.

3. The production method for a cancer examination reagent set according to claim 2,

wherein the (c) is the following (c-1),
(c-1) excluding a part or all of the CpG sites selected for the CpG site set 1, from the CpG sites constituting the CpG site set P and selecting a part or all of remaining CpG sites to create each of ${}_nC_2$ kinds of CpG site sets 2 for discriminating two kinds among the n kinds of cancers.

4. The production method for a cancer examination reagent set according to claim 2 or 3, wherein the number of CpG sites constituting the CpG site set P is 100 or more.

5. A cancer examination method using the cancer examination reagent set according to claim 1, the cancer examination method comprising the following (1) to (3):

(1) a primary examination for examining n kinds of cancers using the primary examination reagent, where n is a natural number of 3 or more;
(2) selecting, in descending order of degree of certainty, two kinds of cancers detected in the primary examination; and
(3) a secondary examination for discriminating the two kinds of cancers using the secondary examination reagent.

**Patentansprüche**

1. Krebsuntersuchungs-Reagenzset, umfassend:

das folgende Primäruntersuchungsreagenz; und
das folgende Sekundäruntersuchungsreagenz,
das Primäruntersuchungsreagenz: Primer, die spezifisch zum Analysieren eines Methylierungsgrads von jeder von allen CpG-Stellen, die einen für Untersuchung von n Arten von Krebs ausgewählten CpG-Stellensatz 1 bilden, ausgelegt sind, wobei n eine natürliche Zahl von 3 oder mehr ist, wobei der CpG-Stellensatz 1 in Tabelle 1 gezeigt ist, wobei die Positionen die Positionen auf den Koordinaten von Genome Reference Consortium Human Build 37 sind:

Tabelle 1

| CpG-Stellensatz 1 | | | |
|---|---|---|---|
| Chromosom | Position | Chromosom | Position |
| 21 | 36399258 | 1 | 14026590 |
| 10 | 97802941 | 2 | 37899953 |
| 7 | 73508618 | 7 | 27196302 |
| 8 | 55370423 | 2 | 144695078 |
| 19 | 57831909 | 10 | 122708532 |
| 16 | 67034882 | 10 | 113943887 |
| 16 | 58498190 | 7 | 96634660 |
| 5 | 169724656 | 9 | 124461171 |
| 12 | 117798954 | 14 | 23356251 |
| 12 | 105478345 | 7 | 27196365 |
| 1 | 78511856 | 5 | 43007632 |
| 3 | 142682652 | 9 | 130212635 |
| 11 | 67351271 | 7 | 16794078 |
| 1 | 29586414 | 14 | 37126902 |
| 1 | 143913847 | 6 | 152623304 |
| 17 | 61524073 | 1 | 63795711 |
| 15 | 90208810 | 5 | 40681137 |
| 1 | 151810893 | 10 | 102792249 |
| 1 | 29586299 | 5 | 178004204 |
| 6 | 10887047 | 3 | 168864101 |
| 12 | 104850745 | 7 | 42267719 |
| 13 | 98628060 | 16 | 215960 |
| 15 | 76633086 | 7 | 142986693 |
| 7 | 1272545 | 2 | 162280519 |
| 17 | 75369939 | 11 | 66624841 |
| 17 | 36105117 | 11 | 94502658 |
| 6 | 10422322 | 7 | 134832850 |
| 22 | 50623687 | 1 | 1072902 |
| 14 | 52734525 | 16 | 58498585 |
| 17 | 76921845 | 17 | 40825980 |
| | | 3 | 140660335 |

das Sekundäruntersuchungsreagenz: ein Satz von ${}_nC_2$ Arten von Primern, die spezifisch zum Analysieren eines Methylierungsgrads von jeder von allen CpG-Stellen, die ${}_nC_2$ Arten von CpG-Stellensätzen 2 bilden, ausgelegt sind, wobei die ${}_nC_2$ Arten zum jeweiligen Diskriminieren von zwei Arten unter den n Arten von Krebs ausgewählt sind, wobei die CpG-Stellensätze 2 in den folgenden Tabellen 2 bis 11 gezeigt sind, wobei die Positionen die Positionen auf den Koordinaten von Genome Reference Consortium Human Build 37 sind:

Tabelle 2

| CpG-Stellensatz 2 von Darmkrebs/Bauchspeicheldrüsenkrebs | | | |
|---|---|---|---|
| Chromosom | Position | Chromosom | Position |
| 16 | 31228059 | 2 | 74425763 |
| 2 | 264164 | 20 | 4803300 |
| 1 | 113497999 | 11 | 33850605 |
| 6 | 43142093 | 19 | 57862627 |
| 2 | 29338432 | 1 | 143913409 |
| 7 | 96650192 | 5 | 38557085 |
| 14 | 52734529 | 3 | 47029335 |
| 19 | 56904901 | 7 | 134143823 |
| 2 | 74425749 | 6 | 30652399 |
| 1 | 35258594 | 2 | 29338121 |
| 17 | 36734911 | 10 | 135072960 |
| 1 | 63795934 | 7 | 27196153 |
| 8 | 105235606 | 12 | 6665370 |
| 1 | 113498106 | 19 | 10406145 |
| 7 | 27205230 | 19 | 57831678 |
| 2 | 216877984 | 18 | 12407806 |
| 4 | 46391159 | 4 | 188916726 |
| 17 | 75370611 | 12 | 15374303 |
| 16 | 58497767 | 13 | 36920660 |
| 2 | 177043255 | 14 | 24803925 |
| 14 | 102554977 | 2 | 242157117 |
| 8 | 72756341 | 4 | 157997178 |
| 20 | 25062447 | 19 | 15090242 |
| 1 | 151810887 | 16 | 69760928 |
| 5 | 528580 | 7 | 27195918 |
| 6 | 27778076 | 6 | 108145539 |

Tabelle 3

| CpG-Stellensatz 2 von Darmkrebs/Leberzellenkrebs | | | |
|---|---|---|---|
| Chromosom | Position | Chromosom | Position |
| 7 | 50344724 | 6 | 33244976 |
| 13 | 24844861 | 7 | 69064801 |
| 12 | 53142542 | 2 | 63283967 |
| 20 | 2781262 | 2 | 74425593 |
| 3 | 142839578 | 1 | 98519504 |
| 5 | 146258195 | 3 | 9993818 |
| 13 | 28498384 | 2 | 174219336 |
| 2 | 100938903 | 19 | 57862638 |
| 14 | 100438440 | 6 | 43044771 |
| 7 | 27225528 | 7 | 30722320 |
| 3 | 142682682 | 7 | 27194614 |
| 2 | 66667433 | 19 | 58220494 |
| 4 | 13539099 | 15 | 72612125 |
| 7 | 94284678 | 2 | 264166 |
| 19 | 3434945 | 22 | 38808815 |
| 7 | 1272515 | 15 | 68260574 |
| 7 | 27205262 | 5 | 16180076 |
| 6 | 28303932 | 3 | 101497982 |
| 9 | 36258600 | 5 | 112073398 |
| 9 | 27528432 | 22 | 32026873 |
| 17 | 75462189 | 19 | 43979614 |
| 20 | 50248076 | 14 | 105714589 |
| 4 | 7194519 | 3 | 9904411 |
| | | 6 | 28304088 |

Tabelle 4

| CpG-Stellensatz 2 von Darmkrebs/Gallengangkrebs | | | |
|---|---|---|---|
| Chromosom | Position | Chromosom | Position |
| 3 | 44803293 | 20 | 25062447 |
| 1 | 14026584 | 12 | 28128669 |
| 20 | 56324074 | 7 | 42267747 |
| 7 | 21582758 | 17 | 46806056 |
| 1 | 247495818 | 8 | 145013249 |
| 20 | 19192675 | 12 | 24715538 |
| 3 | 50402563 | 7 | 27205224 |
| 11 | 415111 | 10 | 102588691 |
| 17 | 35297146 | 7 | 27196296 |
| 17 | 75369219 | 10 | 17271994 |
| 2 | 56151139 | 12 | 25055967 |
| 10 | 124895441 | 7 | 155167038 |
| 1 | 33219687 | 17 | 38347603 |
| 19 | 57831909 | 3 | 181421427 |
| 6 | 43142093 | 3 | 9993818 |
| 7 | 96650192 | 6 | 43044771 |
| 19 | 56904901 | 1 | 151810589 |
| 15 | 45670478 | 1 | 143913552 |
| 12 | 111472375 | 10 | 21463485 |
| 1 | 113498106 | 3 | 16555379 |
| 6 | 3023894 | 12 | 6665370 |
| 7 | 27205230 | 12 | 24716204 |
| 2 | 216877984 | 19 | 57831678 |
| 11 | 79151188 | 12 | 15374303 |
| 5 | 141031122 | 2 | 216878510 |
| 5 | 74231171 | 19 | 47776728 |

Tabelle 5

| CpG-Stellensatz 2 von Darmkrebs/Eierstockkrebs | | | |
|---|---|---|---|
| Chromosom | Position | Chromosom | Position |
| 13 | 26625307 | 6 | 10391412 |
| 16 | 31228059 | 10 | 11206838 |
| 5 | 16180048 | 7 | 19157193 |
| 16 | 23193848 | 19 | 57862480 |
| 7 | 96650192 | 14 | 52734325 |
| 2 | 176993632 | 1 | 145395753 |
| 6 | 28304249 | 10 | 17271944 |
| 5 | 115152494 | 10 | 102588691 |
| 3 | 119041385 | 7 | 27196296 |
| 3 | 101497980 | 8 | 104383722 |
| 1 | 61548783 | 5 | 43019873 |
| 7 | 31375861 | 3 | 181421427 |
| 12 | 111472375 | 7 | 100318190 |
| 10 | 11206870 | 12 | 96883381 |
| 10 | 88730946 | 14 | 51027861 |
| 2 | 236579780 | 2 | 63283967 |
| 10 | 93393030 | 2 | 219736312 |
| 11 | 79151188 | 17 | 42287971 |
| 4 | 184644321 | 10 | 135072960 |
| 7 | 64349133 | 7 | 27196153 |
| 15 | 79382995 | 10 | 17270431 |
| 19 | 57831816 | 10 | 97802966 |
| 7 | 27196314 | 3 | 49757016 |
| 13 | 32605218 | 4 | 157997178 |
| 12 | 28128669 | 2 | 264204 |
| 7 | 42267747 | 7 | 27204981 |
| 14 | 55596356 | 3 | 13114797 |

Tabelle 6

| CpG-Stellensatz 2 von Bauchspeicheldrüsenkrebs/Leberzellenkrebs | | | |
|---|---|---|---|
| Chromosom | Position | Chromosom | Position |
| 13 | 50070550 | 4 | 156297858 |
| 14 | 105715025 | 15 | 78556940 |
| 1 | 7764737 | 4 | 5710372 |
| 2 | 162283705 | 12 | 111471202 |
| 1 | 46998715 | 17 | 29297873 |
| 17 | 53343283 | 1 | 31845959 |
| 12 | 98897187 | 13 | 107186870 |
| 12 | 25055262 | 12 | 25055967 |
| 4 | 76556042 | 22 | 38808815 |
| 5 | 54468707 | 15 | 68260574 |
| 5 | 178004204 | 5 | 112073398 |
| 1 | 7692367 | 11 | 2905931 |
| 3 | 141516291 | 14 | 105714589 |
| 14 | 100259329 | 6 | 152958133 |
| 3 | 119041529 | 1 | 151812435 |
| 15 | 34807221 | 1 | 183440909 |
| 19 | 11492376 | 5 | 102091873 |
| 4 | 156298050 | 2 | 242973936 |
| 6 | 28304075 | 11 | 13030676 |
| 14 | 105512306 | 2 | 74782096 |
| 13 | 41187926 | 10 | 11206868 |
| 3 | 40428643 | 11 | 70303464 |
| 17 | 73083812 | 7 | 138720803 |
| 19 | 38755287 | 7 | 128470913 |
| | | 6 | 41606439 |

Tabelle 7

| CpG-Stellensatz 2 von Darmkrebs/Gallengangkrebs | | | |
|---|---|---|---|
| Chromosom | Position | Chromosom | Position |
| 16 | 31228059 | 11 | 70601781 |
| 9 | 71789653 | 6 | 30139634 |
| 10 | 119256257 | 1 | 235116731 |
| 6 | 39304008 | 1 | 2222253 |
| 2 | 223176167 | 19 | 57862612 |
| 10 | 71078141 | 5 | 16180072 |
| 12 | 25055262 | 5 | 115152326 |
| 17 | 75369228 | 4 | 5710372 |
| 14 | 105940391 | 3 | 16555379 |
| 2 | 264164 | 12 | 24716204 |
| 5 | 141395447 | 1 | 156893792 |
| 17 | 4802847 | 6 | 73332073 |
| 2 | 29338432 | 4 | 159092553 |
| 13 | 41187926 | 2 | 219736167 |
| 14 | 52734529 | 8 | 55370336 |
| 19 | 56904901 | 1 | 3663902 |
| 8 | 72756155 | 19 | 47776728 |
| 5 | 141031125 | 19 | 37096148 |
| 1 | 63795934 | 16 | 69760928 |
| 11 | 119227105 | 1 | 29586418 |
| 5 | 178487382 | 19 | 30019529 |
| 2 | 239072674 | 19 | 38754930 |
| 19 | 43979464 | 6 | 108145539 |

Tabelle 8

| CpG-Stellensatz 2 von Darmkrebs/Eierstockkrebs | | | |
|---|---|---|---|
| Chromosom | Position | Chromosom | Position |
| 16 | 58497395 | 12 | 111472375 |
| 7 | 28449474 | 2 | 236579780 |
| 19 | 11450198 | 3 | 193587780 |
| 16 | 89641296 | 10 | 106028628 |
| 7 | 27206544 | 13 | 32605218 |
| 1 | 219347458 | 10 | 114136073 |
| 12 | 111471192 | 6 | 10391412 |
| 11 | 13690160 | 19 | 57862480 |
| 2 | 264164 | 8 | 16884549 |
| 12 | 24715484 | 14 | 23356059 |
| 1 | 119530600 | 14 | 55595768 |
| 2 | 31456747 | 6 | 33043574 |
| 1 | 21616641 | 4 | 53728510 |
| 17 | 75368902 | 12 | 25056243 |
| 2 | 219736549 | 1 | 151811620 |
| 1 | 182584520 | 17 | 44897431 |
| 16 | 23193848 | 2 | 219736167 |
| 2 | 74426422 | 20 | 37434552 |
| 1 | 236558465 | 8 | 38411708 |
| 2 | 66666470 | 2 | 31456964 |
| 12 | 54088972 | 15 | 45670865 |
| 6 | 28304249 | 11 | 13690157 |
| 1 | 154297848 | 2 | 131721099 |
| 4 | 76555777 | 12 | 111618936 |
| 7 | 27196555 | 1 | 29586418 |
| 12 | 25102072 | 5 | 40681893 |
| 11 | 128564874 | 3 | 13114797 |
| 10 | 11207907 | 1 | 38513245 |

Tabelle 9

| CpG-Stellensatz 2 von Leberzellenkrebs/Gallengangkrebs | | | |
|---|---|---|---|
| Chromosom | Position | Chromosom | Position |
| 14 | 105940179 | 17 | 75369055 |
| 17 | 53343283 | 14 | 36003826 |
| 12 | 98897187 | 15 | 78556940 |
| 4 | 76556042 | 12 | 111471202 |
| 5 | 54468707 | 8 | 145013249 |
| 19 | 56904945 | 19 | 3435350 |
| 7 | 50348174 | 2 | 131130103 |
| 11 | 507455 | 2 | 127414108 |
| 11 | 14927004 | 1 | 12123262 |
| 6 | 73331680 | 7 | 27205159 |
| 22 | 44577135 | 11 | 830320 |
| 7 | 100488942 | 8 | 67874178 |
| 14 | 100259329 | 2 | 264166 |
| 3 | 119041529 | 22 | 38808815 |
| 6 | 42739021 | 5 | 112073398 |
| 4 | 126237421 | 9 | 36258171 |
| 3 | 119041385 | 11 | 2905931 |
| 2 | 239072674 | 19 | 43979614 |
| 7 | 157361692 | 1 | 156405438 |
| 6 | 3023894 | 12 | 25056243 |
| 11 | 79151188 | 1 | 151811620 |
| 3 | 40428643 | 4 | 76555634 |
| 7 | 138720786 | 6 | 42739049 |
| 2 | 100938813 | 12 | 133065941 |
| 19 | 38755287 | 10 | 114134915 |
| 4 | 156297858 | 2 | 242973936 |
|  |  | 2 | 119606746 |

Tabelle 10

| CpG-Stellensatz 2 von Leberzellenkrebs/Eierstockkrebs | | | |
|---|---|---|---|
| Chromosom | Position | Chromosom | Position |
| 5 | 54468707 | 13 | 32605218 |
| 19 | 56904945 | 1 | 145395753 |
| 7 | 50348174 | 5 | 177540239 |
| 14 | 105940391 | 1 | 31845959 |
| 6 | 73331680 | 8 | 16884549 |
| 17 | 4802847 | 2 | 131130103 |
| 4 | 156298050 | 5 | 162931471 |
| 16 | 27437900 | 12 | 25055967 |
| 16 | 23193848 | 8 | 104383722 |
| 14 | 105512306 | 8 | 67874178 |
| 2 | 74426422 | 15 | 60296981 |
| 5 | 141031147 | 7 | 27205658 |
| 7 | 27196320 | 9 | 36258171 |
| 1 | 236558465 | 13 | 32605406 |
| 1 | 154297848 | 1 | 43390705 |
| 11 | 119227105 | 8 | 27183097 |
| 12 | 25102072 | 3 | 16555379 |
| 22 | 50623692 | 10 | 17270431 |
| 3 | 101497980 | 4 | 76555634 |
| 1 | 156051324 | 6 | 42739049 |
| 10 | 11207907 | 12 | 133065941 |
| 19 | 43979464 | 19 | 47776728 |
| 10 | 11206813 | 5 | 176831364 |
| 7 | 27195602 | 7 | 27204981 |
| 3 | 193587780 | 16 | 46783018 |
| 10 | 106028628 | 1 | 29586418 |
| 3 | 40428643 | 16 | 85932666 |
| 5 | 16180072 | 19 | 38754930 |
| | | 6 | 108145539 |

Tabelle 11

| CpG-Stellensatz 2 von Gallengangkrebs/Eierstockkrebs | | | |
|---|---|---|---|
| Chromosom | Position | Chromosom | Position |
| 14 | 64805784 | 13 | 88324597 |
| 9 | 71789653 | 1 | 236558465 |
| 16 | 58498151 | 14 | 52734529 |
| 7 | 73624319 | 12 | 54088972 |
| 19 | 30016147 | 15 | 72612720 |
| 11 | 44590889 | 6 | 28304249 |
| 6 | 39304008 | 1 | 154297848 |
| 10 | 71078141 | 4 | 76555777 |
| 7 | 27206544 | 7 | 27196555 |
| 12 | 124246919 | 12 | 25102072 |
| 17 | 75369228 | 19 | 43979464 |
| 6 | 150358985 | 6 | 30140018 |
| 1 | 219347458 | 14 | 60618622 |
| 12 | 111471192 | 15 | 83953880 |
| 19 | 1467952 | 14 | 52734325 |
| 11 | 13690702 | 1 | 145395753 |
| 13 | 36920813 | 14 | 23356059 |
| 1 | 24645917 | 5 | 162931471 |
| 2 | 31456747 | 3 | 16555379 |
| 1 | 182584520 | 10 | 17270431 |
| 17 | 4802847 | 1 | 151811620 |
| 6 | 130339617 | 19 | 47776728 |
| 16 | 23193848 | 1 | 29586418 |
| | | 1 | 38513245 |

**2.** Produktionsverfahren für ein Krebsuntersuchungs-Reagenzset, das ein Primäruntersuchungsreagenz und ein Sekundäruntersuchungsreagenz enthält, wobei das Produktionsverfahren für ein Krebsuntersuchungs-Reagenzset die folgenden (a) bis (e) umfasst:

(a) Erzeugen eines CpG-Stellensatzes P, der aus CpG-Stellen besteht, die einen signifikanten Unterschied in einem Methylierungsgrad bei mindestens einer Art unter n Arten von Krebs aufweisen, wobei n eine natürliche Zahl von 3 oder mehr ist;
(b) Auswählen eines Teils der CpG-Stellen, die den CpG-Stellensatz P bilden, um einen CpG-Stellensatz 1 zu erzeugen;
(c) Auswählen eines Teils der CpG-Stellen, die den CpG-Stellensatz P bilden, um jede von ${}_{n}C_{2}$ Arten von CpG-Stellensätzen 2 zum Diskriminieren von zwei Arten unter den n Arten von Krebs zu erzeugen;
(d) Produzieren von Primern, die spezifisch zum Analysieren eines Methylierungsgrads des CpG-Stellensatzes 1 ausgelegt sind, um das Primäruntersuchungsreagenz zu bilden; und
(e) Produzieren von ${}_{n}C_{2}$ Arten von Primern, die jeweils spezifisch zum Analysieren von Methylierungsgraden der ${}_{n}C_{2}$ Arten von CpG-Stellensätzen 2 ausgelegt sind, um das Sekundäruntersuchungsreagenz zu bilden.

**3.** Produktionsverfahren für ein Krebsuntersuchungs-Reagenzset nach Anspruch 2,

wobei das (c) das folgende (c-1) ist,
(c-1) Ausschließen eines Teils oder aller der CpG-Stellen, die für den CpG-Stellensatz 1 ausgewählt worden sind, aus den CpG-Stellen, die den CpG-Stellensatz P bilden, und Auswählen eines Teils oder aller verbleibenden CpG-Stellen, um jede von ${}_{n}C_{2}$ Arten von CpG-Stellensätzen 2 zum Diskriminieren von zwei Arten unter den n Arten von Krebs zu erzeugen.

**4.** Produktionsverfahren für ein Krebsuntersuchungs-Reagenzset nach Anspruch 2 oder 3, wobei die Anzahl an CpG-Stellen, die den CpG-Stellensatz P bilden, 100 oder mehr beträgt.

**5.** Krebsuntersuchungsverfahren unter Verwendung des Krebsuntersuchungs-Reagenzsets nach Anspruch 1, wobei das Krebsuntersuchungsverfahren die folgenden (1) bis (3) umfasst:

(1) eine Primäruntersuchung zum Untersuchen von n Arten von Krebs unter Verwendung des Primäruntersuchungsreagenzes, wobei n eine natürliche Zahl von 3 oder mehr ist;
(2) Auswählen von zwei Arten von Krebs, die bei der Primäruntersuchung detektiert worden sind, in absteigender Reihenfolge von Gewissheitsgrad; und
(3) eine Sekundäruntersuchung zum Diskriminieren der zwei Arten von Krebs unter Verwendung des Sekundäruntersuchungsreagenzes.

## Revendications

**1.** Ensemble de réactifs d'examen du cancer comprenant :

le réactif d'examen primaire suivant ; et
le réactif d'examen secondaire suivant,
le réactif d'examen primaire : des amorces spécifiquement conçues pour analyser un degré de méthylation de chacun de tous les sites CpG constituant un ensemble de sites CpG 1 sélectionné pour examiner n types de cancers, où n est un nombre naturel de 3 ou plus, dans lequel l'ensemble de sites CpG 1 est présenté dans le tableau 1, dans lequel les positions sont les positions sur les coordonnées de Genome Reference Consortium Human Build 37 :

Tableau 1

| Ensemble de sites CpG 1 | | | |
|---|---|---|---|
| Chromosome | Position | Chromosome | Position |
| 21 | 36399258 | 1 | 14026590 |
| 10 | 97802941 | 2 | 37899953 |
| 7 | 73508618 | 7 | 27196302 |
| 8 | 55370423 | 2 | 144695078 |
| 19 | 57831909 | 10 | 122708532 |
| 16 | 67034882 | 10 | 113943887 |
| 16 | 58498190 | 7 | 96634660 |
| 5 | 169724656 | 9 | 124461171 |
| 12 | 117798954 | 14 | 23356251 |
| 12 | 105478345 | 7 | 27196365 |
| 1 | 78511856 | 5 | 43007632 |
| 3 | 142682652 | 9 | 130212635 |
| 11 | 67351271 | 7 | 16794078 |
| 1 | 29586414 | 14 | 37126902 |
| 1 | 143913847 | 6 | 152623304 |
| 17 | 61524073 | 1 | 63795711 |
| 15 | 90208810 | 5 | 40681137 |
| 1 | 151810893 | 10 | 102792249 |
| 1 | 29586299 | 5 | 178004204 |
| 6 | 10887047 | 3 | 168864101 |
| 12 | 104850745 | 7 | 42267719 |
| 13 | 98628060 | 16 | 215960 |
| 15 | 76633086 | 7 | 142986693 |
| 7 | 1272545 | 2 | 162280519 |
| 17 | 75369939 | 11 | 66624841 |
| 17 | 36105117 | 11 | 94502658 |
| 6 | 10422322 | 7 | 134832850 |
| 22 | 50623687 | 1 | 1072902 |
| 14 | 52734525 | 16 | 58498585 |
| 17 | 76921845 | 17 | 40825980 |
| | | 3 | 140660335 |

le réactif d'examen secondaire : un ensemble de ${}_nC_2$ types d'amorces spécifiquement conçues pour analyser un degré de méthylation de chacun de tous les sites CpG constituant ${}_nC_2$ types d'ensembles de sites CpG 2, les ${}_nC_2$

types étant sélectionnés pour discriminer respectivement deux types parmi les n types de cancers, dans lequel les ensembles de sites CpG 2 sont présentés dans les tableaux 2 à 11 suivants, dans lequel les positions sont les positions sur les coordonnées de Genome Reference Consortium Human Build 37 :

Tableau 2

| Ensemble de sites CpG 2 de cancer du côlon/cancer du pancréas | | | |
|---|---|---|---|
| Chromosome | Position | Chromosome | Position |
| 16 | 31228059 | 2 | 74425763 |
| 2 | 264164 | 20 | 4803300 |
| 1 | 113497999 | 11 | 33850605 |
| 6 | 43142093 | 19 | 57862627 |
| 2 | 29338432 | 1 | 143913409 |
| 7 | 96650192 | 5 | 38557085 |
| 14 | 52734529 | 3 | 47029335 |
| 19 | 56904901 | 7 | 134143823 |
| 2 | 74425749 | 6 | 30652399 |
| 1 | 35258594 | 2 | 29338121 |
| 17 | 36734911 | 10 | 135072960 |
| 1 | 63795934 | 7 | 27196153 |
| 8 | 105235606 | 12 | 6665370 |
| 1 | 113498106 | 19 | 10406145 |
| 7 | 27205230 | 19 | 57831678 |
| 2 | 216877984 | 18 | 12407806 |
| 4 | 46391159 | 4 | 188916726 |
| 17 | 75370611 | 12 | 15374303 |
| 16 | 58497767 | 13 | 36920660 |
| 2 | 177043255 | 14 | 24803925 |
| 14 | 102554977 | 2 | 242157117 |
| 8 | 72756341 | 4 | 157997178 |
| 20 | 25062447 | 19 | 15090242 |
| 1 | 151810887 | 16 | 69760928 |
| 5 | 528580 | 7 | 27195918 |
| 6 | 27778076 | 6 | 108145539 |

Tableau 3

| Ensemble de sites CpG 2 de cancer du côlon/cancer hépatocellulaire | | | |
|---|---|---|---|
| Chromosome | Position | Chromosome | Position |
| 7 | 50344724 | 6 | 33244976 |
| 13 | 24844861 | 7 | 69064801 |
| 12 | 53142542 | 2 | 63283967 |
| 20 | 2781262 | 2 | 74425593 |
| 3 | 142839578 | 1 | 98519504 |
| 5 | 146258195 | 3 | 9993818 |
| 13 | 28498384 | 2 | 174219336 |
| 2 | 100938903 | 19 | 57862638 |
| 14 | 100438440 | 6 | 43044771 |
| 7 | 27225528 | 7 | 30722320 |
| 3 | 142682682 | 7 | 27194614 |
| 2 | 66667433 | 19 | 58220494 |
| 4 | 13539099 | 15 | 72612125 |
| 7 | 94284678 | 2 | 264166 |
| 19 | 3434945 | 22 | 38808815 |
| 7 | 1272515 | 15 | 68260574 |
| 7 | 27205262 | 5 | 16180076 |
| 6 | 28303932 | 3 | 101497982 |
| 9 | 36258600 | 5 | 112073398 |
| 9 | 27528432 | 22 | 32026873 |
| 17 | 75462189 | 19 | 43979614 |
| 20 | 50248076 | 14 | 105714589 |
| 4 | 7194519 | 3 | 9904411 |
| | | 6 | 28304088 |

Tableau 4

| Ensemble de sites CpG2 de cancer du côlon/cancer des voies biliaires | | | | |
|---|---|---|---|---|
| Chromosome | Position | | Chromosome | Position |
| 3 | 44803293 | | 20 | 25062447 |
| 1 | 14026584 | | 12 | 28128669 |
| 20 | 56324074 | | 7 | 42267747 |
| 7 | 21582758 | | 17 | 46806056 |
| 1 | 247495818 | | 8 | 145013249 |
| 20 | 19192675 | | 12 | 24715538 |
| 3 | 50402563 | | 7 | 27205224 |
| 11 | 415111 | | 10 | 102588691 |
| 17 | 35297146 | | 7 | 27196296 |
| 17 | 75369219 | | 10 | 17271994 |
| 2 | 56151139 | | 12 | 25055967 |
| 10 | 124895441 | | 7 | 155167038 |
| 1 | 33219687 | | 17 | 38347603 |
| 19 | 57831909 | | 3 | 181421427 |
| 6 | 43142093 | | 3 | 9993818 |
| 7 | 96650192 | | 6 | 43044771 |
| 19 | 56904901 | | 1 | 151810589 |
| 15 | 45670478 | | 1 | 143913552 |
| 12 | 111472375 | | 10 | 21463485 |
| 1 | 113498106 | | 3 | 16555379 |
| 6 | 3023894 | | 12 | 6665370 |
| 7 | 27205230 | | 12 | 24716204 |
| 2 | 216877984 | | 19 | 57831678 |
| 11 | 79151188 | | 12 | 15374303 |
| 5 | 141031122 | | 2 | 216878510 |
| 5 | 74231171 | | 19 | 47776728 |

Tableau 5

| Ensemble de sites CpG 2 de cancer du côlon/cancer ovarien | | | |
|---|---|---|---|
| Chromosome | Position | Chromosome | Position |
| 13 | 26625307 | 6 | 10391412 |
| 16 | 31228059 | 10 | 11206838 |
| 5 | 16180048 | 7 | 19157193 |
| 16 | 23193848 | 19 | 57862480 |
| 7 | 96650192 | 14 | 52734325 |
| 2 | 176993632 | 1 | 145395753 |
| 6 | 28304249 | 10 | 17271944 |
| 5 | 115152494 | 10 | 102588691 |
| 3 | 119041385 | 7 | 27196296 |
| 3 | 101497980 | 8 | 104383722 |
| 1 | 61548783 | 5 | 43019873 |
| 7 | 31375861 | 3 | 181421427 |
| 12 | 111472375 | 7 | 100318190 |
| 10 | 11206870 | 12 | 96883381 |
| 10 | 88730946 | 14 | 51027861 |
| 2 | 236579780 | 2 | 63283967 |
| 10 | 93393030 | 2 | 219736312 |
| 11 | 79151188 | 17 | 42287971 |
| 4 | 184644321 | 10 | 135072960 |
| 7 | 64349133 | 7 | 27196153 |
| 15 | 79382995 | 10 | 17270431 |
| 19 | 57831816 | 10 | 97802966 |
| 7 | 27196314 | 3 | 49757016 |
| 13 | 32605218 | 4 | 157997178 |
| 12 | 28128669 | 2 | 264204 |
| 7 | 42267747 | 7 | 27204981 |
| 14 | 55596356 | 3 | 13114797 |

Tableau 6

| Ensemble de sites CpG 2 de cancer du pancréas/cancer hépatocellulaire | | | |
|---|---|---|---|
| Chromosome | Position | Chromosome | Position |
| 13 | 50070550 | 4 | 156297858 |
| 14 | 105715025 | 15 | 78556940 |
| 1 | 7764737 | 4 | 5710372 |
| 2 | 162283705 | 12 | 111471202 |
| 1 | 46998715 | 17 | 29297873 |
| 17 | 53343283 | 1 | 31845959 |
| 12 | 98897187 | 13 | 107186870 |
| 12 | 25055262 | 12 | 25055967 |
| 4 | 76556042 | 22 | 38808815 |
| 5 | 54468707 | 15 | 68260574 |
| 5 | 178004204 | 5 | 112073398 |
| 1 | 7692367 | 11 | 2905931 |
| 3 | 141516291 | 14 | 105714589 |
| 14 | 100259329 | 6 | 152958133 |
| 3 | 119041529 | 1 | 151812435 |
| 15 | 34807221 | 1 | 183440909 |
| 19 | 11492376 | 5 | 102091873 |
| 4 | 156298050 | 2 | 242973936 |
| 6 | 28304075 | 11 | 13030676 |
| 14 | 105512306 | 2 | 74782096 |
| 13 | 41187926 | 10 | 11206868 |
| 3 | 40428643 | 11 | 70303464 |
| 17 | 73083812 | 7 | 138720803 |
| 19 | 38755287 | 7 | 128470913 |
| | | 6 | 41606439 |

Tableau 7

| Ensemble de sites CpG 2 de cancer du côlon/cancer des voies biliaires | | | |
|---|---|---|---|
| Chromosome | Position | Chromosome | Position |
| 16 | 31228059 | 11 | 70601781 |
| 9 | 71789653 | 6 | 30139634 |
| 10 | 119256257 | 1 | 235116731 |
| 6 | 39304008 | 1 | 2222253 |
| 2 | 223176167 | 19 | 57862612 |
| 10 | 71078141 | 5 | 16180072 |
| 12 | 25055262 | 5 | 115152326 |
| 17 | 75369228 | 4 | 5710372 |
| 14 | 105940391 | 3 | 16555379 |
| 2 | 264164 | 12 | 24716204 |
| 5 | 141395447 | 1 | 156893792 |
| 17 | 4802847 | 6 | 73332073 |
| 2 | 29338432 | 4 | 159092553 |
| 13 | 41187926 | 2 | 219736167 |
| 14 | 52734529 | 8 | 55370336 |
| 19 | 56904901 | 1 | 3663902 |
| 8 | 72756155 | 19 | 47776728 |
| 5 | 141031125 | 19 | 37096148 |
| 1 | 63795934 | 16 | 69760928 |
| 11 | 119227105 | 1 | 29586418 |
| 5 | 178487382 | 19 | 30019529 |
| 2 | 239072674 | 19 | 38754930 |
| 19 | 43979464 | 6 | 108145539 |

Tableau 8

| Ensemble de sites CpG 2 de cancer du côlon/cancer ovarien | | | |
|---|---|---|---|
| Chromosome | Position | Chromosome | Position |
| 16 | 58497395 | 12 | 111472375 |
| 7 | 28449474 | 2 | 236579780 |
| 19 | 11450198 | 3 | 193587780 |
| 16 | 89641296 | 10 | 106028628 |
| 7 | 27206544 | 13 | 32605218 |
| 1 | 219347458 | 10 | 114136073 |
| 12 | 111471192 | 6 | 10391412 |
| 11 | 13690160 | 19 | 57862480 |
| 2 | 264164 | 8 | 16884549 |
| 12 | 24715484 | 14 | 23356059 |
| 1 | 119530600 | 14 | 55595768 |
| 2 | 31456747 | 6 | 33043574 |
| 1 | 21616641 | 4 | 53728510 |
| 17 | 75368902 | 12 | 25056243 |
| 2 | 219736549 | 1 | 151811620 |
| 1 | 182584520 | 17 | 44897431 |
| 16 | 23193848 | 2 | 219736167 |
| 2 | 74426422 | 20 | 37434552 |
| 1 | 236558465 | 8 | 38411708 |
| 2 | 66666470 | 2 | 31456964 |
| 12 | 54088972 | 15 | 45670865 |
| 6 | 28304249 | 11 | 13690157 |
| 1 | 154297848 | 2 | 131721099 |
| 4 | 76555777 | 12 | 111618936 |
| 7 | 27196555 | 1 | 29586418 |
| 12 | 25102072 | 5 | 40681893 |
| 11 | 128564874 | 3 | 13114797 |
| 10 | 11207907 | 1 | 38513245 |

Tableau 9

| Ensemble de sites CpG 2 de cancer hépatocellulaire/cancer des voies biliaires | | | |
|---|---|---|---|
| Chromosome | Position | Chromosome | Position |
| 14 | 105940179 | 17 | 75369055 |
| 17 | 53343283 | 14 | 36003826 |
| 12 | 98897187 | 15 | 78556940 |
| 4 | 76556042 | 12 | 111471202 |
| 5 | 54468707 | 8 | 145013249 |
| 19 | 56904945 | 19 | 3435350 |
| 7 | 50348174 | 2 | 131130103 |
| 11 | 507455 | 2 | 127414108 |
| 11 | 14927004 | 1 | 12123262 |
| 6 | 73331680 | 7 | 27205159 |
| 22 | 44577135 | 11 | 830320 |
| 7 | 100488942 | 8 | 67874178 |
| 14 | 100259329 | 2 | 264166 |
| 3 | 119041529 | 22 | 38808815 |
| 6 | 42739021 | 5 | 112073398 |
| 4 | 126237421 | 9 | 36258171 |
| 3 | 119041385 | 11 | 2905931 |
| 2 | 239072674 | 19 | 43979614 |
| 7 | 157361692 | 1 | 156405438 |
| 6 | 3023894 | 12 | 25056243 |
| 11 | 79151188 | 1 | 151811620 |
| 3 | 40428643 | 4 | 76555634 |
| 7 | 138720786 | 6 | 42739049 |
| 2 | 100938813 | 12 | 133065941 |
| 19 | 38755287 | 10 | 114134915 |
| 4 | 156297858 | 2 | 242973936 |
|  |  | 2 | 119606746 |

Tableau 10

| Ensemble de sites CpG 2 de cancer hépatocellulaire/cancer ovarien | | | |
|---|---|---|---|
| Chromosome | Position | Chromosome | Position |
| 5 | 54468707 | 13 | 32605218 |
| 19 | 56904945 | 1 | 145395753 |
| 7 | 50348174 | 5 | 177540239 |
| 14 | 105940391 | 1 | 31845959 |
| 6 | 73331680 | 8 | 16884549 |
| 17 | 4802847 | 2 | 131130103 |
| 4 | 156298050 | 5 | 162931471 |
| 16 | 27437900 | 12 | 25055967 |
| 16 | 23193848 | 8 | 104383722 |
| 14 | 105512306 | 8 | 67874178 |
| 2 | 74426422 | 15 | 60296981 |
| 5 | 141031147 | 7 | 27205658 |
| 7 | 27196320 | 9 | 36258171 |
| 1 | 236558465 | 13 | 32605406 |
| 1 | 154297848 | 1 | 43390705 |
| 11 | 119227105 | 8 | 27183097 |
| 12 | 25102072 | 3 | 16555379 |
| 22 | 50623692 | 10 | 17270431 |
| 3 | 101497980 | 4 | 76555634 |
| 1 | 156051324 | 6 | 42739049 |
| 10 | 11207907 | 12 | 133065941 |
| 19 | 43979464 | 19 | 47776728 |
| 10 | 11206813 | 5 | 176831364 |
| 7 | 27195602 | 7 | 27204981 |
| 3 | 193587780 | 16 | 46783018 |
| 10 | 106028628 | 1 | 29586418 |
| 3 | 40428643 | 16 | 85932666 |
| 5 | 16180072 | 19 | 38754930 |
| | | 6 | 108145539 |

Tableau 11

| Ensemble de sites CpG 2 de cancer des voies biliaires/cancer ovarien | | | |
|---|---|---|---|
| Chromosome | Position | Chromosome | Position |
| 14 | 64805784 | 13 | 88324597 |
| 9 | 71789653 | 1 | 236558465 |
| 16 | 58498151 | 14 | 52734529 |
| 7 | 73624319 | 12 | 54088972 |
| 19 | 30016147 | 15 | 72612720 |
| 11 | 44590889 | 6 | 28304249 |
| 6 | 39304008 | 1 | 154297848 |
| 10 | 71078141 | 4 | 76555777 |
| 7 | 27206544 | 7 | 27196555 |
| 12 | 124246919 | 12 | 25102072 |
| 17 | 75369228 | 19 | 43979464 |
| 6 | 150358985 | 6 | 30140018 |
| 1 | 219347458 | 14 | 60618622 |
| 12 | 111471192 | 15 | 83953880 |
| 19 | 1467952 | 14 | 52734325 |
| 11 | 13690702 | 1 | 145395753 |
| 13 | 36920813 | 14 | 23356059 |
| 1 | 24645917 | 5 | 162931471 |
| 2 | 31456747 | 3 | 16555379 |
| 1 | 182584520 | 10 | 17270431 |
| 17 | 4802847 | 1 | 151811620 |
| 6 | 130339617 | 19 | 47776728 |
| 16 | 23193848 | 1 | 29586418 |
| | | 1 | 38513245 |

2. Procédé de production d'un ensemble de réactifs d'examen du cancer incluant un réactif d'examen primaire et un réactif d'examen secondaire, le procédé de production d'un ensemble de réactifs d'examen du cancer comprenant les étapes (a) à (e) suivantes :

   (a) créer un ensemble de sites CpG P constitué de sites CpG présentant une différence significative de degré de méthylation dans au moins un type parmi n types de cancers, où n est un nombre naturel de 3 ou plus ;
   (b) sélectionner une partie des sites CpG constituant l'ensemble de sites CpG P pour créer un ensemble de sites CpG 1 ;
   (c) sélectionner une partie des sites CpG constituant l'ensemble de sites CpG P pour créer chacun de ${}_nC_2$ types d'ensembles de sites CpG 2 pour discriminer deux types parmi les n types de cancers ;
   (d) produire des amorces spécifiquement conçues pour analyser un degré de méthylation de l'ensemble de sites CpG 1 pour former le réactif d'examen primaire ; et
   (e) produire ${}_nC_2$ types d'amorces spécifiquement conçues pour analyser des degrés de méthylation des ${}_nC_2$ types d'ensembles de sites CpG 2, respectivement, pour former le réactif d'examen secondaire.

3. Procédé de production d'un ensemble de réactifs d'examen du cancer selon la revendication 2,

   dans lequel l'étape (c) est la suivante (c-1),
   (c-1) exclure une partie ou la totalité des sites CpG sélectionnés pour l'ensemble de sites CpG 1 parmi les sites CpG constituant l'ensemble de sites CpG P, et sélectionner une partie ou la totalité des sites CpG restants pour créer chacun de ${}_nC_2$ types d'ensembles de sites CpG 2 pour discriminer deux types parmi les n types de cancers.

4. Procédé de production d'un ensemble de réactifs d'examen du cancer selon la revendication 2 ou la revendication 3, dans lequel le nombre de sites CpG constituant l'ensemble de sites CpG P est de 100 ou plus.

5. Procédé d'examen du cancer utilisant l'ensemble de réactifs d'examen du cancer selon la revendication 1, le procédé d'examen du cancer comprenant les étapes (1) à (3) suivantes :

   (1) un examen primaire pour examiner n types de cancers en utilisant le réactif d'examen primaire, où n est un

nombre naturel de 3 ou plus ;
(2) sélectionner, dans l'ordre décroissant de degré de certitude, deux types de cancers détectés lors de l'examen primaire ; et
(3) un examen secondaire pour discriminer les deux types de cancers en utilisant le réactif d'examen secondaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020096601 A
- JP 2009009396 A
- WO 2020227127 A2
- WO 2020163403 A1
- US 2020255904 A1